# EUROPEAN PATENT SPECIFICATION

(11) **EP 4 249 468 B1**
(45) Date of publication and mention of the grant of the patent: **07.05.2025**
(21) Application number: 22163288.8
(22) Date of filing: 21.03.2022
(51) Int. Cl.: C07C 227/14, C07D 213/18, C07D 233/58, C07C 279/02, C01G 21/16, C07C 209/68, C07C 277/00

(54) **A METHOD FOR SYNTHESIS OF HALIDE SALTS**
VERFAHREN ZUR SYNTHESE VON HALOGENIDSALZEN
PROCÉDÉ DE SYNTHÈSE DE SELS D'HALOGÉNURE

(43) Date of publication of application: 27.09.2023
(73) Proprietor: Solaveni GmbH, 59199 Bönen (DE)
(72) Inventor: Öz, Senol, 51065 Köln (DE); Przypis, Lukasz, 44-186 Gieraltowice (PL)
(74) Representative: Kancelaria Eupatent.pl Sp. z.o.o

(56) References cited:
- EP-A2- 3 719 001
- KHAN YEASIN ET AL: "Waterproof perovskites: high fluorescence quantum yield and stability from a methylammonium lead bromide/formate mixture in water", JOURNAL OF MATERIALS CHEMISTRY C, vol. 8, no. 17, 7 May 2020 (2020-05-07), GB, pages 5873 - 5881, XP055957891, ISSN: 2050-7526, DOI: 10.1039/D0TC00383B

## Description

### TECHNICAL FIELD

The present disclosure relates to a method for synthesis of halide salts.

### BACKGROUND

Among halide salts, there are known organic halide salts, including halide salts of ammonia derivatives as well as inorganic halide salts, including metal-halide salts.

The methods for synthesis of the halide salts, both organic and inorganic, aiming at improving the purity of the obtained products and reducing the consumption of harmful or toxic reagents and solvents, are of constant development. *Inter alia,* the improved purity of the synthesized halide salts can reduce the post-synthesis treatment procedures, such as extraction, precipitation, filtration, charcoal treatment, distillation, etc.

The halide salts of high purity are used in various sectors of industry. For example, inorganic halide salts - such as lead bromide, as well as organic halide salts such as ammonium halide derivatives, e.g., methylammonium iodide (MAI), are used for synthesizing perovskites for photo-active materials used *inter alia* for the production of solar cells.

Moreover, various methods of synthesizing halide salts are known from the patent literature.

A publication o EP application EP3719001 describes a method for synthesis of organic iodides of general formula RₓNI; the method consists in synthesizing hydrogen iodide (HI) *in situ* by mixing molecular iodine with formic acid in the molar ratio of I₂ : COOH no less than 1.01:1; next introducing, into the synthesizes HI, a compound being a donor of organic cation: RₓN⁺, providing the molar ratio of RₓN⁺:I₂ of no less than 1.01:1, and next maintaining the reaction mixture at a temperature of not less than 20°C to obtain the reaction product being the salt with the general formula RₓNI. Synthesizing HI at the first step of the reaction product and maintaining certain ratios of the reactants prevent the formation of by-products being formates of respective organic cations ([RₓN⁺] [HCOO⁻]), whose donors are added to the reaction mixture. This way the obtained product can be purified in a simple, low-cost manner.

A publication of European patent EP2072496 describes a method for obtaining pure halide quaternary ammonium salt by reducing the amount of a protonic acid salt of a tertiary amine in the quaternary ammonium salt. The method consists in adding an oxide or hydroxide of metal to the quaternary ammonium salt contaminated with a protonic acid salt of a tertiary amine, and next removing the tertiary amine and water by distillation or heating while introducing nitrogen, argon or air, and metal salt produced by filtration or column purification.

In the processes for producing the halide salts belonging to the group of quaternary amine derivatives, typically, the synthesis involves reacting an acid with a respective halide. However, in the course of such synthesis, a part of tertiary amine of basis character (an organic basis), can left unreacted, and thereby can react with the acid to produce a protonic acid salt, permitting the acid salt to remain in the main product of quaternary ammonium salt as the contamination. Further, deacidification of the product solutions is necessary since complete removal of organic bases is possible only after said salts of the tertiary amine have been decomposed.

Further, preparing heavy metal halide salts may involve reacting two soluble metal salts, e.g. heavy metal nitrate with alkali metal halide, to form one soluble salt and one insoluble salt with may be removed by precipitation. For instance, lead nitrate solution and potassium iodide solution can react to produce solid lead iodide, leaving soluble potassium nitrate in the solution. However, the precipitate is typically contaminated with the soluble salt, thus, it may require further purification by washing and filtration procedures.

As follows from the above, many attempts have been taken to address the problem with the purity of the synthesized halide salts. However, despite the various approaches of modifying the synthesis pathway, the known methods do not provide the satisfactory effect of purity of the synthesized halide salts, due to incomplete conversion of the substrates, necessity of using certain substrates in excess, or the presence of by-products in the post-reaction mixture. Thereby, the application of labor-intensive and time-consuming post-treatment procedures, which generate additional costs and chemical wastes, are typically required to obtain the halide salts of required purity.

### SUMMARY OF THE INVENTION

There is a need to provide a method for the synthesis of halide salts providing improved purity of the obtained products, thereby, limiting the required post-treating procedures.

In one aspect of the present disclosure, there is provided a method for synthesis of halide salts of the general formula (I):

**Zⁿ⁺X⁻ₙ** **Formula I**

wherein:
X⁻ represents a halogen anion, and
Zⁿ⁺ represents a cation having a valency n,
the method comprising reacting a first reactant being formate salt of the general formula (II):

**Zⁿ⁺(COOH⁻)ₙ** **Formula II**

with a second reactant being a diatom halogen: X₂.

Preferably, said diatom halogen X₂ is selected from Br₂ and I₂.

Preferably, said cation (Zⁿ⁺) is an inorganic cation of a metal selected from the group consisting of Cs⁺, Ag⁺, Pb²⁺, Sn²⁺, Bi³⁺, and Sn⁴⁺.

Preferably, said cation (Zⁿ⁺) is Pb²⁺.

Alternatively preferably, said cation (Zⁿ⁺) is an organic cation of the general formula (III), (IV), or (V): 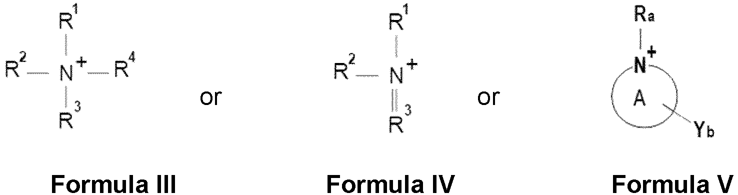 wherein:
**R, R¹, R², R³, R⁴** each independently represents hydrogen or an organic substituent moiety, wherein at least one of R¹, R², R³ is not hydrogen;
**a** denotes the number of R moieties,
**A** represents five-membered or six-membered aliphatic or aromatic hydrocarbon ring, optionally having at least one heteroatom substituting the carbon atom selected from oxygen, nitrogen, and sulphur,
**Y** represents a substituent of the ring A other than hydrogen.
**b** - denotes the number of Y moieties, wherein b is zero or an integer in the range of 1 to 4 if A represents the five-membered ring, or b is zero or an integer in the range of 1 to 5 if A represents the six-membered ring.

Preferably, the organic substituent independently for each of R, R¹, R², R³, and R⁴, is selected from C₁-C₁₆ alkyl, C₆ aryl or C₁₀ aryl, C₇₋₂₆ aralkyl, or heterocycle substituent.

Preferably, the organic substituent independently for each of R, R¹, R², R³, and R⁴, is selected from:
C₁-C₁₆ alkyl constituting acyclic, straight or branched hydrocarbon chain comprising from 1 to 16 carbon atoms in the chain, optionally comprising at least one heteroatom substituting the carbon atom or hydrogen atom in the C₁-C₁₆ alkyl moiety, wherein the heteroatom being selected from the group consisting of phosphorus, silicon, oxygen, nitrogen, sulphur, iodine, chlorine, bromine, and fluorine;
C₆ aryl or C₁₀ aryl constituting an aromatic monocyclic system comprising six carbon atoms forming the ring system, or an aromatic bicyclic system comprising ten carbon atoms forming the bicyclic system, optionally comprising at least one heteroatom substituting the carbon atom or hydrogen atom in the C₆ aryl or C₁₀ aryl moiety, wherein the heteroatom is selected from the group consisting of phosphorus, silicon, oxygen, nitrogen, sulphur, iodine, chlorine, bromine, and fluorine;
C₇₋₂₆ aralkyl constituting the C₆ arlyl or C₁₀ aryl substituent linked to the nitrogen atom of the organic cation through the C₁-C₁₆ alkyl, optionally comprising at least one heteroatom substituting the carbon atom or hydrogen atom, wherein the heteroatom is selected from the group consisting of: phosphorus, silicon, oxygen, nitrogen, sulphur, iodine, chlorine, bromine and fluorine; and
heterocycle substituent constituting monovalent substituent derived by removing hydrogen from a five-, six-, or seven-membered saturated or unsaturated alicyclic moiety or aromatic heterocycle moiety, comprising carbon atoms and from one to four ring heteroatoms selected from nitrogen, oxygen, and sulfur.

Preferably, the organic substituent independently for each of R, R¹, R², R³ and R⁴, is selected from:
C₁-C₁₆ alkyl substituent selected from the group consisting of methyl, ethyl, propyl, isopropyl n-butyl, tert-butyl, isobutyl, sec-butyl, n-pentyl, isopenthyl, neopenthyl, methylethyl, diispropylethyl, 2-methylhexyl, 3-methylheptyl, 2,3-dimethylpentyl, 4-ethyl-2-methyloctyl, 4-isopropylnonyl, hexyl, 2-methylpentyl, 3-methylpentyl, 2,2-dimethylbutyl, and 2,3-dimethylbutyl, 1-methylhexyl, 2-methylhexyl, 1,1-dimethylpentyl, 1,2- dimethylpentyl, 1,3- dimethylpentyl, 2,2-dimethylpentyl, 3-ethylpentyl, 2,2,3-trimethylbutyl, 2-methylheptyl, 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, 2,2,4-trimethylpentyl, 2,3,3-Trimethylpentyl, 3,3,4-trimethyl-pentyl, 3,4,4-trimethylpentyl, 2,4,4-trimethylpentyl, 2-methylheptyl, 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, optionally comprising at least one heteroatom substituting the carbon atom or hydrogen atom in the C₁-C₁₆ alkyl moiety, wherein the heteroatom being selected from the group consisting of phosphorus, silicon, oxygen, nitrogen, sulphur, iodine, chlorine, bromine, and fluorine;
C₆ aryl or C₁₀ aryl substituent selected from the group consisting of phenyl and naphthyl, optionally comprising at least one heteroatom substituting the carbon atom or hydrogen atom in the C₆ or C₁₀ aryl moiety, wherein the heteroatom is selected from the group consisting of: phosphorus, silicon, oxygen, nitrogen, sulphur, iodine, chlorine, bromine, and fluorine;
C₇₋₂₆ aralkyl substituent selected from the group consisting of benzyl, butylphenyl, ethylphenyl, propylphenyl, pentylphenyl, hexylphenyl, ethylnaphtyl, and methylnaphtyl, optionally comprising at least one heteroatom substituting the carbon atom or hydrogen atom, wherein the heteroatom is selected from the group consisting of phosphorus, silicon, oxygen, nitrogen, sulphur, iodine, chlorine, bromine and fluorine; and
heterocycle substituent selected from the group consisting of furan, tetrahydrofuran, thiophene, diazepine, isoxazole, piperidine, dioxane, pyridine, and pyrimidine.

Preferably, said cation (Zⁿ⁺) is the organic cation of the formula (VI), (VII), or (VIII):

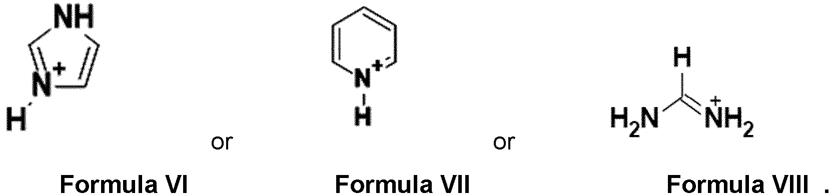

Preferably, the method comprises heating the reactants to the temperature of 25 to 130°C until obtaining the halide salt.

Preferably, reacting of the reactants is carried out in the absence of a solvent.

Alternatively preferably, reacting of the reactants is carried out in the presence of a solvent.

Preferably, the solvent is selected from the group consisting of water, and acetone.

Preferably, the solvent is selected from the group consisting of water, alcohols, and acetonitriles.

Preferably, the method further comprises recrystallizing the obtained halide salt.

Further aspects and features of the present invention are described in the following description of the drawings.

### BRIEF DESCRIPTION OF DRAWINGS

Aspects and features of the present invention will become apparent by describing, in detail, exemplary embodiments of the present invention with reference to the attached drawings, in which:
Fig. 1 presents a reaction of a method for synthesis of halide salts according to the present disclosure;
Fig. 2 presents an embodiment of a reaction of a method for synthesis of halide salts according to the present disclosure;
Fig 3 presents *in situ* formation of formate salt and the following reaction of a method for synthesis of halide salts according to the present disclosure;
Fig. 4 presents 1H-NMR spectrum of methylammonium iodide synthesized according to one of the embodiments;
Fig. 5 presents 13C-NMR spectrum of methylammonium iodide synthesized according to one of the embodiments;
Fig. 6 presents IR spectrum of methylammonium iodide synthesized according to one of the embodiments;
Fig. 7 presents IR spectrum of methylammonium bromide synthesized according to one of the embodiments;
Fig. 8 presents 1H-NMR spectrum of formidinium iodide synthesized according to one of the embodiments;
Fig. 9 presents 13C-NMR spectrum of formamidinium iodide synthesized according to one of the embodiments;
Fig. 10 presents IR spectrum of formamidinium iodide synthesized according to one of the embodiments;
Fig. 11 presents 1H-NMR spectrum of tetramethylammonium iodide synthesized according to one of the embodiments;
Fig. 12 presents 13C-NMR spectrum of tetramethylammonium iodide synthesized according to one of the embodiments;
Fig. 13 presents IR spectrum tetramethylammonium iodide synthesized according to one of the embodiments;
Fig. 14 presents 1H-NMR spectrum triethylammonium iodide synthesized according to one of the embodiments;
Fig. 15 presents 13C-NMR spectrum triethylammonium iodide synthesized according to one of the embodiments;
Fig. 16 presents IR spectrum of triethylammonium iodide synthesized according to one of the embodiments;
Fig. 17 presents 1H-NMR spectrum of imidazolium iodide synthesized according to one of the embodiments;
Fig. 18 presents 13C-NMR spectrum of imidazolium iodide synthesized according to one of the embodiments;
Fig. 19 presents IR spectrum of imidazolium iodide synthesized according to one of the embodiments;
Fig. 20 presents 1H-NMR spectrum of pyridinium iodide synthesized according to one of the embodiments;
Fig. 21 presents 13C-NMR spectrum of pyridinium iodide synthesized according to one of the embodiments;
Fig. 22 presents IR spectrum of pyridinium iodide synthesized according to one of the embodiments;
Fig. 23 presents 1H-NMR spectrum of aniline hydroiodide synthesized according to one of the embodiments;
Fig. 24 presents 13C-NMR spectrum of aniline hydroiodide synthesized according to one of the embodiments;
Fig. 25 presents 1H-NMR spectrum of 3-aminopropionic acid hydroiodide synthesized according to one of the embodiments;
Fig. 26 presents 13C-NMR spectrum of 3-aminopropionic acid hydroiodide synthesized according to one of the embodiments;
Fig. 27 presents IR spectrum of 3-aminopropionic acid hydroiodide synthesized according to one of the embodiments;
Fig. 28 presents 1H-NMR spectrum of N-methylglycine hydroiodide synthesized according to one of the embodiments;
Fig. 29 presents 13C-NMR spectrum of N-methylglycine hydroiodide synthesized according to one of the embodiments;
Fig. 30 presents IR spectrum of N-methylglycine hydroiodide synthesized according to one of the embodiments;
Fig. 31 presents 1H-NMR spectrum of glycine hydroiodide synthesized according to one of the embodiments;
Fig. 32 presents 13C-NMR spectrum of glycine hydroiodide synthesized according to one of the embodiments;
Fig. 33 presents IR spectrum of glycine hydroiodide synthesized according to one of the embodiments;
Fig. 34 presents 1H-NMR spectrum of benzylammonium iodide synthesized according to one of the embodiments;
Fig. 35 presents 13C-NMR spectrum of benzylammonium iodide synthesized according to one of the embodiments;
Fig. 36 presents IR spectrum of benzylammonium iodide synthesized according to one of the embodiments;
Fig. 37 presents X-ray diffraction pattern of PbI2 synthesized according to one of the embodiments;
Fig. 38 presents X-ray diffraction pattern of PbBr2 synthesized according to one of the embodiments;

### DETAILED DESCRIPTION OF EMBODIMENTS

Reference will now be made to embodiments, examples of which are illustrated in the accompanying drawings. Aspects and features of the embodiments will be described with reference to the accompanying drawings. The present invention, however, may be embodied in various different forms and should not be construed as being limited only to the illustrated embodiments. Rather, these embodiments are provided as examples so that this disclosure will be thorough and complete, and will fully convey the aspects and features of the present invention to those skilled in the art. It shall be understood that not all of the features shown in the embodiments are essential and the scope of the protection is defined not by means of literally shown embodiments, but by the features provided in the claims.

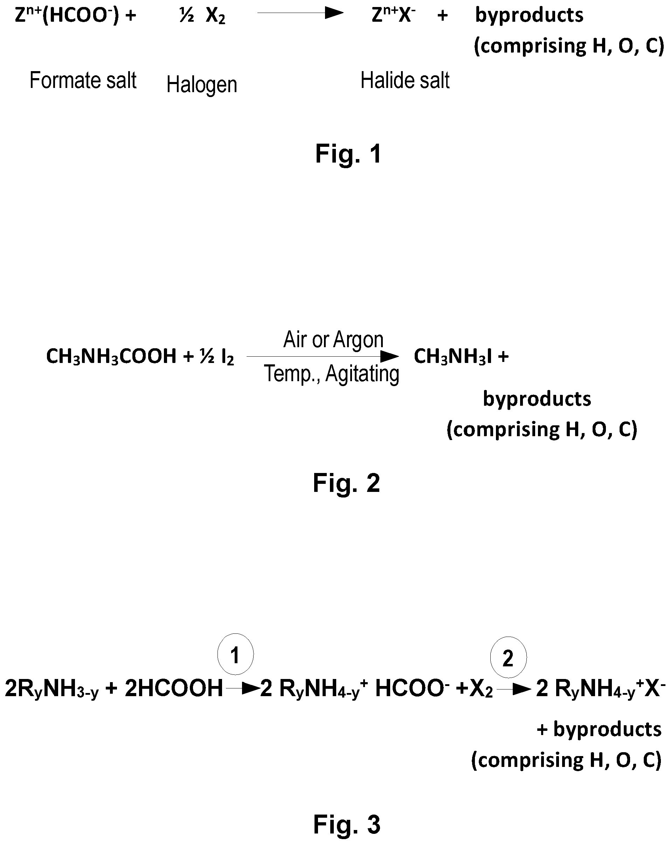

The method for synthesis of halide salts according to the present disclosure involves reacting a formate salt of the general formula Zⁿ⁺(HCOO)ₙ with a diatom halogen: X₂, preferably Br₂ or I₂ as schematically shown in Fig. 1. The formate salt: Zⁿ⁺(COOH⁻)ₙ reduces the molecular (diatom) halide X₂ to ions: X⁻ to form the halide salt of the general formula Znⁿ⁺(X⁻)ₙ, whereas the formate ions: HCOO⁻ transform to gaseous and/or liquid byproducts comprising C, O and/or H. It is suspected that the reaction by products comprises CO and/or CO₂, and H2O and/or H2, and more likely H₂O. However, due to the volatile nature of the byproducts the chemical formulas of the byproducts are under investigation.

The reaction may occur with providing the stoichiometric ratio of the substrates. However, if one of the substrates, either the formate salt (Zⁿ⁺(COOH⁻)ₙ) or the halide (X₂) is provided in excess, the reaction proceeds until total consumption of the other - deficient substrate. Thereby, no particular limitation for a ratio of these two substrates is required to carry out the synthesis of the halide salts, e.g. the halogen X₂ can be added in the amount ranging from 0,5 to 1,2 molar equivalents of the formate salt Zⁿ⁺(HCOO)ₙ.

To maintain a reasonable reaction time, preferably the reactants: Zⁿ⁺(COOH⁻)ₙ and X₂ are heated to the temperature of at least 25°C, and preferably to the temperature of at least 60°C or more, and more preferably the reactants are heated to a temperature between 25 and 130°C, and preferably between 60 and 130°C. For example, the mixture of Zⁿ⁺(COOH⁻)ₙ and X₂ can be heated to a temperature such as 25°C, 30°C, 40°C, 50°C, 60°C, 70°C, 75°C, 80°C, 85°C, 90°C, or 95° C, and most preferably 70° C, to obtain the halide salt. During heating the mixture of reactants: Zⁿ⁺(COOH⁻)ₙ and X₂ can be agitated, e.g. stirred or shacked - to provide an increased level of molecules interactions. However, in the case of smaller amounts of the reactants, e.g., in the order of milligrams/milliliters, agitation is not necessarily required. Agitation of the reactants is preferably carried out throughout all the reaction duration. Agitating can be preferably performed from a few seconds to a few hours, and preferably from 1 minute to 4 hours, e.g., from 30 minutes to 1 hour.

Furthermore, the optimum synthesis conditions and times of the reaction may vary depending on the reactants used. Unless otherwise specified, solvents or solvent-free conditions, temperatures, pressures, and other reaction conditions may be readily selected by one of ordinary skill in the art to obtain optimum results for a particular reaction. Optionally, the reaction product may be purified by re-crystallization. For instance, the halide salt - being the reaction product can be obtained in pure form, in liquid or solid state - depending on its melting point, by simple removal of a reaction media such as solvent - if present, under reduced pressure and temperature below boiling or sublimation or decomposition point of the obtained halide salt. If the substrates are used in the stoichiometric ratio, both the substrates can be consumed in total, thereby, not contaminating the product, which enables one to achieved improved purity of the obtained halide salt.

In the course of the reaction, no acids such as hydrohalic acids (HX) are synthesized - the pH value of the reaction media, throughout the reaction of formate salt with the halide (X₂), remains neutral, ca. pH = 7. It is believed that the reaction of the formate salt with the halide: X₂ occurs by a free radical mechanism, and the halide: X₂ does not interact with protons H⁺ e.g. supplied with the solvent; it is further suspected that the reduction of halide: X₂ by formate salt constitutes a reaction that is privileged notwithstanding the solvent used, because the reaction leads to the halide salts in the solvent-free conditions as well. Is it further suspected that the solvent molecules, if present in the reaction medium, do not interact with the reactants forming intermediate products or transitional states (such as low-energy transitional states).

### Substrates and Products

The method for synthesis according to the present disclosure allows obtaining halide salts according to the general formula (I):

**Zⁿ⁺X⁻ₙ** **Formula I**

wherein:
Zⁿ⁺ represents a cation being either inorganic cation - preferably the cation of metal, or organic cation, preferably being ammonium derivative having the positive charge substantially localized on the nitrogen atom; n stands for valency of the cation Zⁿ⁺, n preferably equals 1, 2, 3 or 4; it will be readily apparent from the description of the present disclosure that for the organic cations typically n = 1, whereby for the inorganic cations n may vary e.g. n = 1, 2, 3 or 4.
X⁻ - is a halogen ion, preferably Br⁻ or I⁻.

### Organic Cation Zⁿ⁺

Preferably, the organic cation Zⁿ⁺ of the halide salt is a derivative of amine, being either aliphatic amine, aromatic amine, aliphatic-aromatic amine, or aryloamine, including, where applicable, primary amine, secondary amine, tertiary amine; or derivative of imine such as e.g., formamidinium cation. The halide salt is obtained from the corresponding formate salt, comprising said organic cation Zⁿ⁺, as shown schematically in Fig. 1.

Preferably, the organic cation (Zⁿ⁺) is represented by the general formula (III), (IV), or (V): 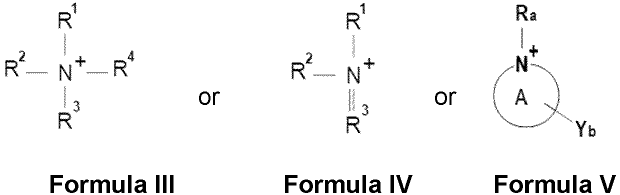 wherein
**R, R¹, R², R³, R⁴** each independently represents hydrogen or an organic substituent, with the proviso that at least one of R¹, R², R³ is not hydrogen;
a - denotes the number of R moieties at nitrogen atom, **a** may equal 1 or 2 - so as to provide N⁺;
**A** represents a five-membered or six-membered aliphatic or aromatic hydrocarbon ring, optionally having at least one heteroatom substituting the carbon atom, the heteroatom is preferably selected from oxygen, nitrogen, and sulphur.
**Y** represents a substituent, other than hydrogen, being the substituent at the ring A, wherein Y is preferably a chemically inert group, i.e. the group that is not able to chemically interfere with the formation of the formate salt or with the final reaction product, i.e. the halide salt. Preferably, the substituent Y is selected from the group consisting of alkyl, aryl, carboxy, alkoxy, halogen, thiol, hydroxy, trihaloalkyl, and (per)haloalkyl.
**b** - denotes the number of Y moieties, wherein b is zero or an integer in the range of 1 to 4 if A represents a five-membered ring, or b is zero or an integer in the range of 1 to 5 if A represents six-membered ring.

As mentioned above **R, R¹, R², R³, R⁴** each independently represents hydrogen or an organic substituent, wherein at least one of R¹, R², R³ is not hydrogen; the organic substituent, independently for each of R, R¹, R², R³, and R⁴, is preferably selected from:
- C₁-C₁₆ hydrocarbon substituent constituting straight or branched C₁-C₁₆ alkyl substituent. The term "C₁-C₁₆ alkyl" as used herein, either alone or in combination with other the organic substituent, denotes acyclic, straight or branched chain alkyl substituent comprising from 1 to 16 carbon atoms in the chain; for example, the C₁-C₁₆ alkyl substituent may be selected from the group consisting of methyl, ethyl, propyl, isopropyl, n-butyl, tert-butyl, isobutyl, sec-butyl, n-pentyl, isopenthyl, neopenthyl, methylethyl, diispropylethyl, 2-methylhexyl, 3-methylheptyl, 2,3-dimethylpentyl, 4-ethyl-2-methyloctyl, 4-isopropylnonyl, hexyl, 2-methylpentyl, 3-methylpentyl, 2,2-dimethylbutyl, 2,3-dimethylbutyl, 1-methylhexyl, 2-methylhexyl, dimethylpentyl (including 4 isomers, such as 2,2-dimethylpentyl), 3-ethylpentyl, 2,2,3-trimethylbutyl, 2-methylheptyl, 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, 2,2,4-trimethylpentyl, 2,3,3-trimethylpentyl, 3,3,4-trimethyl-pentyl, 3,4,4-trimethylpentyl, 2,4,4-trimethylpentyl, 2-methylheptyl (including 5 isomers, such as 2-methylhept-1-yl. 2-methylhept-2-yl etc), 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, 2,2,4-trimethylpentyl, 2,3,3-trimethylpentyl, 3,3,4-trimethyl-pentyl, 3,4,4-trimethylpentyl, and 2,4,4-trimethylpentyl (including 5 isomers, such as 2,4,4-trimethylpent-1-yl, 2,4,4-trimethylpent-2-yl). Optionally, the C₁-C₁₆ alkyl can comprise at least one heteroatom, and preferably from 1 to 6 heteroatoms, where applicable, substituting the carbon atom or hydrogen atom in the C₁-C₁₆ alkyl moiety, wherein the heteroatom is selected from the group consisting of phosphorus, silicon, oxygen, nitrogen, sulphur, iodine, chlorine, bromine, and fluorine; no-limiting examples of the C₁-C₁₆ alkyl comprising at least one heteroatom are: 2,2,2-trifluorethylamin, 2,2,2-trichlororethylamin, tris(perfluoropropyl)amine. Optionally the C₁-C₁₆ alkyl can be further substituted with one or more than one functional group, such as amino, carboxy, and/or methoxy;
- C₆ arylor C₁₀ aryl substituent. The term "C₆ aryl or C₁₀ aryl" as used herein, either alone or in combination with other the organic substituent, denotes either an aromatic monocyclic system comprising six carbon atoms forming a ring system or an aromatic bicyclic system containing ten carbon atoms forming bicyclic system; for example, C₆ aryl or C₁₀ aryl substituents may be selected from the group consisting of phenyl and naphthyl. Optionally, the C₆ aryl or C₁₀ aryl can comprise at least one heteroatom, and preferably from 1 to 6 heteroatoms, where applicable, substituting the carbon atom or hydrogen atom in the C₆ aryl or C₁₀ aryl moiety, wherein the heteroatom is selected from the group consisting of phosphorus, silicon, oxygen, nitrogen, sulphur, iodine, chlorine, bromine, and fluorine; non-limiting examples of C₆ aryl or C₁₀ aryl comprising at least one heteroatom are: 4-chlorophenylamine, 2-aminothiophenol, and 4-fluorophenylamine;
- C₇₋₂₆ aralkyl substituent. The term "C₇₋₂₆ aralkyl" as used herein, either alone or in combination with other the organic substituent, denotes the aryl moiety - as defined above, that is linked (substituted) to the nitrogen atom of the cation through the alkyl moiety, wherein the term "alkyl" is as defined above; for example, C₇₋₂₇ aralkyl substituents may be selected from the group consisting of benzyl andbutylphenyl. Optionally, the C₇₋₂₆ aralkyl can comprise at least one heteroatom, and preferably from 1 to 6 heteroatoms, where applicable, substituting the carbon atom or hydrogen atom in the C₇₋₂₆ aralkyl moiety, wherein the heteroatom is selected from the group consisting of phosphorus, silicon, oxygen, nitrogen, sulphur, iodine, chlorine, bromine and fluorine; non-limiting example of C₇₋₂₆ aralkyl comprising at least one heteroatom is 4-fluorophenylethyl-amine;
- heterocycle substituent. The term "heterocycle" as used herein, either alone or in combination with other the organic substituent, means a monovalent substituent derived by removing a hydrogen from a five-, six-, or seven-membered saturated or unsaturated heterocycle, including aromatic heterocycle; the heterocycle moiety comprises carbon atoms and from one to four ring heteroatoms selected from nitrogen, oxygen, and sulfur; for example, the heterocycle substituent may be selected from the group consisting of furan, tetrahydrofuran, thiophene, diazepine, isoxazole, piperidine, dioxane, pyridine, and pyrimidine.

Non-limiting examples of the organic cation Zⁿ⁺ used in the composition of the formic salt Zⁿ⁺(HCOO⁻) to obtain the halide salt Zⁿ⁺(X⁻), according to the present invention (Fig. 1), include: ammonium cations, including alkylammonium cations, dialkylammonium cations, and trialkylammonium cations, amidinium cations, formamidinium cations; for instance Zⁿ⁺ can denote: methylammonium CH₃(H₃)N⁺, ethylammonium CH₃CH₂(H₃)N⁺, propylammonium C₃H₇(H₃)N⁺, butylammonium C₄H₉(H₃)N⁺, pentylammonium H₃C₅H₁₁N⁺, hexylammonium H₃C₆H₁₃N⁺, heptylammonium C₇H₁₅(H₃)N⁺ or octylammonium C₈H₁₇(H₃)N⁺ cation, formamidinium (H₂N-HC=NH₂)⁺, guanidinium (H₂N)₂-C=NH₂)⁺ cation, or acetamidinium cation (H₂N-H₃CC=NH₂)⁺.

Further, the non-limiting examples of the organic cation Zⁿ⁺ include the cations represented by the following formula (VI), (VII), or (VIII):

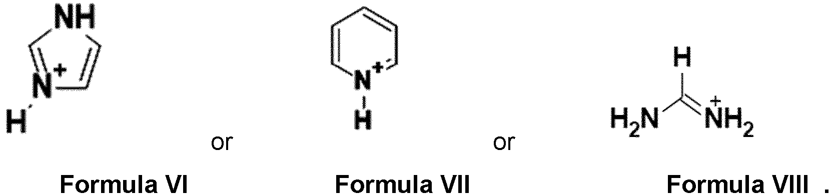

Thus, the developed method involving the reaction shown in Fig. 1 enables one to obtain halide salts, including bromide salts and iodide salts, depending on the halide used: Br₂, I₂, such as methylammonium halide, dimethylammonium halide, propylammonium halide, methylethylammonium halide, butylammonium halide, pentylammonium halide, hexylammonium halide, heptylammonium halide, octylammonium halide formamidinium halide, guanidinium halide, acetamidinium halide, benzylammonium halide, benzyl- methyl ammonium halide, phenethylammonium halide, N,N-dimethylpropane-1,3-diammonium halide, N,N-diethylethane-1,2-diammonium halide, iso-butylammonium halide, isopropylammonium halide, morpholinium halide, phenylammonium halide, dimethylammonium halide, trimethyl ammonium hailde, tertramethylammonium halide, di-isopropylammonium halide, hexane-1,6-diammonium halide, triewthyl ammonium halide, t-butylammonium halide, 4-tertbutyl-phenylammonium halide, butane-1,4-diammonium halide, propane-1,3-diammonium halide, ethane-1,2-diammonium halide, diethylamine halide, N,N-Diethylethane-1,2-diammonium halide, cyclohexylethylammonium halide, 2-(phenylene)di(etyylammonium) halide, 4-t-butylbenzylammonium halide, 2-thiophenmethylammonium halide, 2-(4-Fluorophenyl)ethylammonium halide, 4-Fluorobenzylammonium halide, 5-aminovaleric acid halide, cyclohexanemethylammonium halide, 2-(4-methoxyphenyl)ethylammonium halide, neopentylammonium halide, 1,4-diazabicyclo[2.2.2]octane halide, N,N-dimethylethylenediamine dihalide, dibutylammonium halide, N,N-diethyl-1,3-propanediamine dihalide, piperazinium dihalide, pyrrolidinium halide, 1,4-phenylenediammonium dihalide, 5-azoniaspiro[4.4]nonane halide, cyclohexyl ammonium halide, 4-(trifluoromethyl)benzylammonium halide, 2,4,4-trimethylpentan-2-ammonium halide, N,N-dimethyl-1,3-propanediammmonium dihalide, dodecylammonium halide, dimethylammonium halide, dethylammonium halide, 1-hexyl-1,4-diazabicyclo[2.2.2]octan-1-ium halide, morpholinium halide, n-octlyammonium halide, 4-hydroxyphenethylammonium halide, 4-chlorophenylammonium halide, 4-methoxyphenethylammonium halide, 2-furanmethylammonium halide, biphenylammonium halide, triphenylammonium halide, 2-thiophenethylammonium halide, diethanolammonium halide, ethanolammonium halide, diphenylammonium halide2,2-dipmethylpropane-1,3 diammonium halide, methoxyethylammonium halide, 2-pyrrolidin-1-ium-1-ylethylammonium halide, quinuclidine-1-ium halide, piperidinium halide, tert-octylammonium halide, etc.

### Inorganic Cation Zⁿ⁺

Preferably, the inorganic cation Zⁿ⁺ of the halide salt constitutes a cation of metal Meⁿ⁺, such as Me⁺, Me²⁺, Me³⁺ and Me⁴⁺. Non-limiting examples of the inorganic cation Zⁿ⁺ used as the component of the formic salt Zⁿ⁺(HCOO⁻) - to obtain the halide salt Zⁿ⁺(X⁻) according to the present invention (Fig. 1), include the inorganic cations selected from the group consisting of: Cs⁺, Ag⁺, Pb²⁺, Sn²⁺, Bi³⁺, and Sn⁴⁺. More preferably the inorganic cation is Pb²⁺.

Thus, the developed method involving the reaction shown in Fig. 1 enables one to obtain halide salts, including bromide salts and iodide salts, depending on the halide used: Br₂, I₂, such as cesium halide (CsX), silver halide (AgX), lead (II) halide (PbX₂), tin (II) halide (SnX₂), bismuth (III) halide (BiX₃) and tin (IV) halide (SnX₄), including: CsI, CsBr, AgI, AgBr, PbBr₂, PbI₂, SnBr₂, SnI₂, BiBr₃, BiI₃ SnBr₄, SnI₄.

### Solvents

The reaction, depending on the nature of the substrates, can be carried out in solvent-free conditions, for example for the respective formates (Zⁿ⁺(HCOO⁻)ₙ) being liquids in ambient conditions. Fig. 2 presents the embodiment of synthesis the halide salt, carried out in solvent-free conditions. The methylammonium formate - liquid at room temperature, reacts with iodine (I₂) (solid), in the atmosphere of air or under inert gas (e.g. argon), under heating to a temperature of 25-130 °C and agitating, in solvent-free conditions. The obtained product can constitute pure methylammonium iodide crystals - in the case of using the stoichiometric ratio of the substrates - due to the total consumption of both the substrates, in solvent-free conditions, however, if the molar ratio of the substrates used is non-stoichiometric, e.g. 1: 1 then the reaction can be carried out until total consumption of the deficient substrate. This embodiment thereby indicates that the solvent molecules are not necessarily needed to conduct the reaction. Similar to the above-described reaction of halide with methylammonium formate - the ethylammonium formate, propylammonium formate, butylammonium formate, or pentylammonium formate can be subjected to the reaction.

Alternatively, the reaction can be carried out in a solvent environment - for example for the respective formates (Zⁿ⁺(HCOO⁻)ₙ) being solids in ambient conditions, the solvent allows interacting one reactant (Zⁿ⁺(HCOO⁻)ₙ) with the other reactant (X₂) - as the molcules of the substrates can move in the solvent. Also, it is a common practice to conduct a chemical reaction in a solvent medium, i.a. to improve agitating the mixture of reactants and provide better heat transfer within the reaction chamber. Thereby, the solvent aims at providing respective solubility of the reactants to improve the amounts of molecules interactions to better the product yield. The non-limiting examples of the solvents that can be used according to the present disclosure include polar solvents, non-polar solvents, being either protic or a-protic, organic or inorganic compounds. Further, a mixture of two or more solvent components can be used as the solvent. Non-limiting exemplary embodiments of the solvent, respectively inorganic and organic are water, methanol, ethanol, isopropanol, ethers, e.g. tetrahydrofuran or acetonitrile, or any mixture thereof, such as ethanol-water mixture, ethanol-ether mixture, isopropanol-water mixture, or acetonitrile-water mixture. E.g. alcoholic solvents can be used in pure form or in the form of their mixture with water, e.g. in form of water-alcohol azeotropes, such as a mixture of 95.63 wt% ethanol and 4.37wt% water. Also, substantially pure ethanol can be used as the organic polar solvent. The amount of solvent used is not particularly limited. For example, the amount of the solvent may range between 5 to 30 parts by volume (v/W), and preferably amount of the solvent may range between 6 to 8 parts by volume (v/W).

The purpose of solvent is to provide desired dissolution of at least one of the reactants, whereby the other reactant (undissolved) remains suspended in the reaction mixture, or to provide the desired dissolution of both the reactants. The use of the solvent thereby allows for desired agitation of the reactants to facilitate their chemical interaction.

The respective formate: (Zⁿ⁺(HCOO⁻)ₙ) for synthesis the corresponding halide salt, can be supplied to a reaction chamber in a form of commercially available products, such as, e.g. commercially available ammonium formate, formamidinium formate methylammonium formate. Alternatively, the respective formate: (Zⁿ⁺(HCOO⁻)ₙ) can be prepared *in situ* (in the reaction chamber), e.g., by using known methods. The exemplary scheme for the synthesis of halide salt comprising the organic cation according to the present disclosure, involving *in situ* preparation of the salt of formic acid is schematically shown in Fig. 3. The formula R_{y}NH_{3-y} represents amine compound, which can be e.g., methylamine, which reacts with formic acid (Reaction 1) leading to the respective salt formate: R_{y}NH_{3-y}⁺HCOO⁻, such as e.g. methylammonium formate. Following the synthesis of the formate salt, respective halide X₂ is added to the reaction chamber (Reaction 2) resulting in halide salt. Reaction 1 can be carried out in water - as the solvent. Alternatively, other solvents can be used such as polar/non-polar organic solvent; or Reaction 1 can be carried out in solvent-free conditions.

Reaction 2 can be carried out in solvent-free conditions as well. If both reactions 1 and 2 are conducted as solvent-free, the obtained halide salt is already crystalline; this can be done preferably if one of the substrates is liquid in ambient conditions, or in the temperature of reaction. Furthermore, if the solvent is present, either in Reaction 1 or in Reaction 2, or both reactions, the obtained halide salt, can be obtained by crystallizing the post-reaction mixture.

The orders in Reaction 1 have no particular limitation. For example, the primary/secondary/tertiary/quaternary amine can be reacted with formic acid in the organic or inorganic polar or non-polar solvent. The amount of the amine in Reaction 1 ranges from 0.1 molar equivalents to 2.0 molar equivalents with respect to the formic acid. Furthermore, the ammonium formate salts can be isolated in a liquid or solid state. The addition of formic acid to the amine is preferably performed at a temperature ranging between -40°C to 80°C, in order to avoid condensation reaction with loss of water and amide formation. The said polar organic or inorganic solvent in Reaction 1 is preferably water, methanol, ethanol, isopropanol, tetrahydrofuran or acetonitrile, and more preferably water. The amount of the said organic solvent is not particularly limited, e.g., it can range between 5 to 30 parts by volume (v/W), such as 6 to 8 parts by volume.

Further, halogen (X₂) in Reaction 2 can be added preferably in the amount ranging between 0.5 to 1.2molar equivalents of the formate salt, for example of 0.5 to 1 molar equivalents. The temperature in the reaction chamber during Reaction 2 is preferably maintained in the range of 60°C to 130° C, for example, the temperature of Reaction 2 can be maintained at 60°C, 70°C, 75°C, 80°C, 85°C, 90°C, or 95°C, and more preferably 70°C. During Reaction 2 the reactants are agitated. The duration of Reaction 2, depending on the given formate salt, may range, e.g,. from 1 minute to 4 hours, for example, 30 minutes to 1 hour. The obtained halide salt can be obtained in pure form in liquid or solid state by simple removal of the reaction media, such as solvent, under reduced pressure and temperature below boiling or sublimation or decomposition point of the obtained halide salt.

### EMBODIMENT 1 - synthesis of methylammonium iodide (starting from methylamine):

**CH₃NH₃⁺ I⁻**

As a start, 25.0 ml of formic acid was added slowly under cooling (10 °C) to 56.1 ml of methylamine (40 wt% in H₂O). The resulting solution was heated to 40 °C and 82.3 g of iodine (I₂) was added gradually. Afterward, the product was heated to 110 °C for the time necessary to remove the water. The formed solid was taken up with a 50 ml of hot ethanol and subsequently precipitated in an excess of cold diethyl ether. Afterward, crystals were collected by filtration; the obtained crystals were washed with diethyl ether, and dried in vacuum oven at 60° C, to yield 85.79 g of methylammonium iodide (83% yield).

¹H NMR (300 MHz, DMSO-d6), d: 2,37 (s,3H) 7.49 (s, 3H). ¹³C NMR (75 MHz, DMSO-d6), d: 25.00 (ppm).

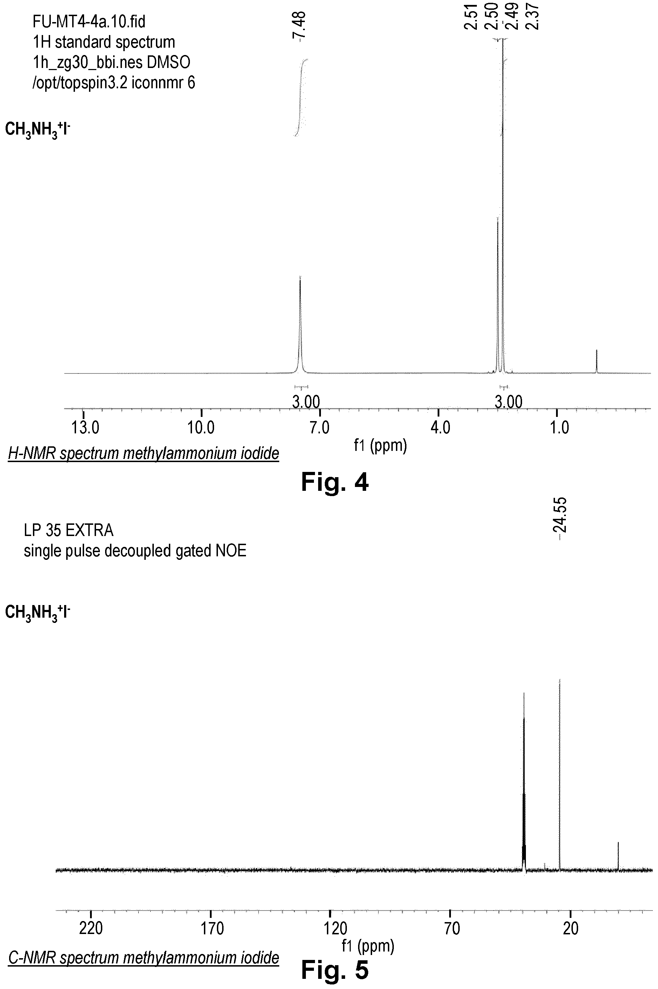

Figs 4, 5 present respective spectra of the obtained porduct.

### EMBODIMENT 2 - synthesis of methylammonium iodide (starting from the isolated ionic liquid methylammonium formate):

**CH₃NH₃⁺ I⁻**

6 g of methylammonium formate was heated at 100°C. Under vigorous stirring, 9.9 g of iodine (I₂) was added gradually. The following precipitate was dissolved in small amounts of hot ethanol and subsequently precipitated in an excess of cold diethyl ether. Afterwards, the crystals were collected by filtration; the crystals were washed with diethyl ether and dried in vacuum oven at 60°C to yield 11 g of methylammonium iodide (89% yield).

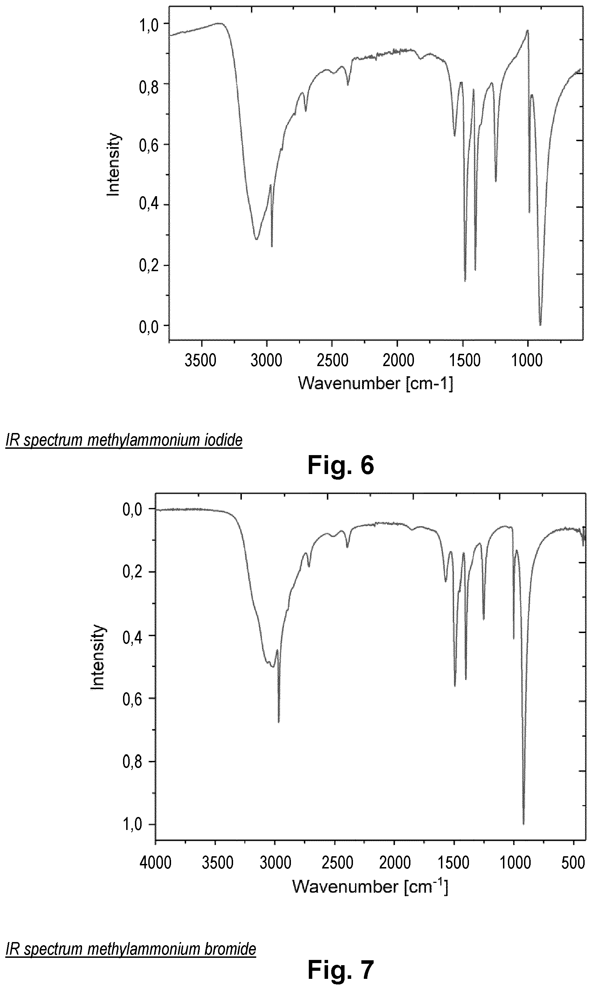

Fig. 6, presents the respective spectrum of the obtained product

### EMBODIMENT 3 - synthesis of methylammonium bromide (starting from methylamine):

**CH₃NH₃⁺ Br⁻**

6.86 ml of formic acid was added slowly under cooling (10 °C) to 15.46 ml of methylamine (40 wt% in H₂O). The resulting solution was further cooled and 1.47 ml (4.6 g) of bromine (Br₂) was added gradually. Afterwards the product was heated to 110 °C to remove the water. The formed solid was redissolved with a few 50 ml of hot ethanol and subsequently precipitated in an excess of cold diethyl ether. Afterward, crystals were collected by filtration; the crystals were washed with diethyl ether, and dried in vacuum oven at 60° C, to yield 15.82 g of methylammonium bromide (79 % yield). ¹H NMR (300 MHz, DMSO-d6), (s, 3H). ¹³C NMR (75 MHz, DMSO-d6) (ppm).

Fig. 7 presents IR spectrum of the obtained product (methylammonium bromide).

### EMBODIMENT 4 - synthesis of methylammonium bromide (starting from the isolated ionic liquid methylammonium formate):

**CH₃NH₃⁺ Br⁻**

6 mL of methylammonium formate was cooled at 10°C. Under vigorous stirring, 2 mL of bromine (Br₂) were slowly added. The following precipitate was dissolved in small amounts of hot ethanol and subsequently precipitated in an excess of cold diethyl ether. Afterwards, the crystals were collected by filtration; the crystals were washed with diethyl ether and dried in vacuum oven at 60°C to yield 7.2 g of methylammonium bromide (83% yield).

### EMBODIMENT 5 - synthesis of formamidinium iodide (starting from formamidine acetate):

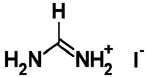

5.0 ml of formic acid was added to 6.57 g of formamidine acetate at room temperature. After the solid was completely dissolved, 8.0 g of iodine (I₂) was added gradually. Afterwards the product was heated to 120 °C to remove the water. The formed solid was redissolved with 50 ml of hot ethanol and subsequently precipitated in an excess of cold diethyl ether. Afterward, crystals were collected by filtration; the crystals were washed with diethyl ether, and dried in vacuum oven at 60° C, to yield 7.28 g of formamidinium iodide (91 % yield).

¹H NMR (300 MHz, DMSO-d6), d: ---- (d, 4H) ----(quint, 1H). ¹³C NMR (75 MHz, DMSO-d6), d (ppm).

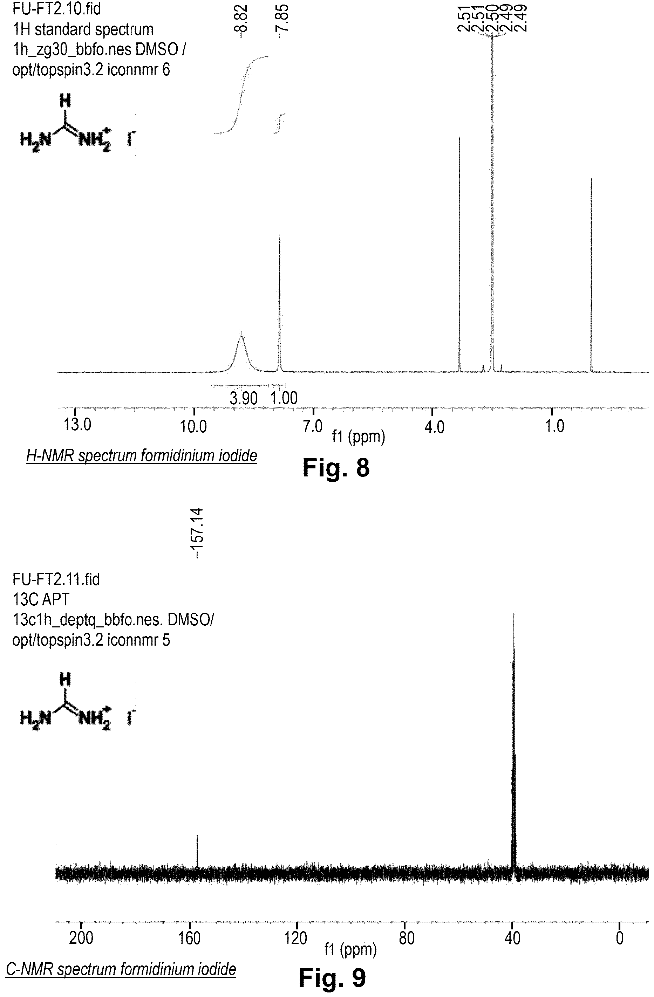

Figs 8, 9, 10 present respective spectra of the obtained product (formamidinium iodide).

### EMBODIMENT 6 - synthesis of formamidinium iodide (starting from isolated ionic liquid formamidine formate):

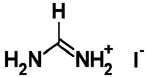

10 g of formamidine formate was heated at 100°C. Under vigorous stirring, 13.5 g of iodine (I₂) was added gradually. The following precipitate was dissolved in small amounts of hot ethanol and subsequently precipitated in an excess of cold diethyl ether. Afterwards, the crystals were collected by filtration; the crystals were washed with diethyl ether and dried in vacuum oven at 60°C to yield 14.7 g of formamidinium iodide (80% yield).

### EMBODIMENT 7 - synthesis of tetramethylammonium iodide (starting from of tetramethylammonium hydroxide):

**(CH₃)₄N⁺ I⁻**

4.80 g of tetramethylammonium hydroxide pentahydrate was weighed and dissolved in 8.0 ml of water. At 10° C, 1.0 ml of formic acid was added into the solution first. Reaction was left at 25 °C for 10 hours. Afterward, 3.36 g of iodine was added. The mixture was agitated for 12 hours at 70° C. The reaction solution was cooled to 25° C. Afterward, crystals as products were collected by filtration, washed with diethyl ether, and air-dried at 50° C, to afforded 5.01 g of title compound (94% yield).

¹H NMR (600 MHz, DMSO-d6), d: 3.20 (s, 12H). ¹³C NMR (150 MHz, D2O), d: 55.85. d (ppm).

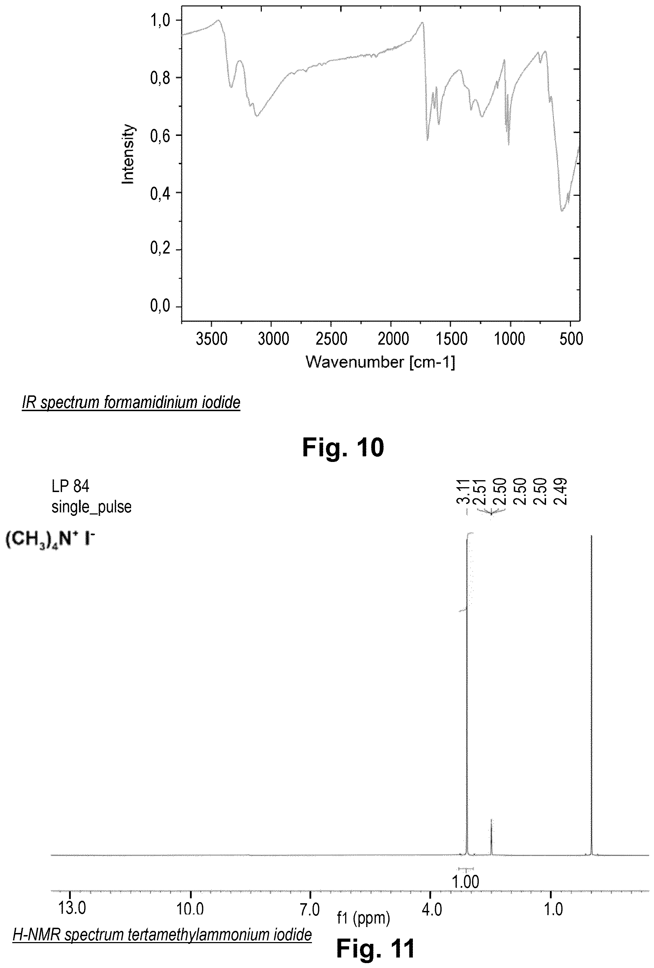

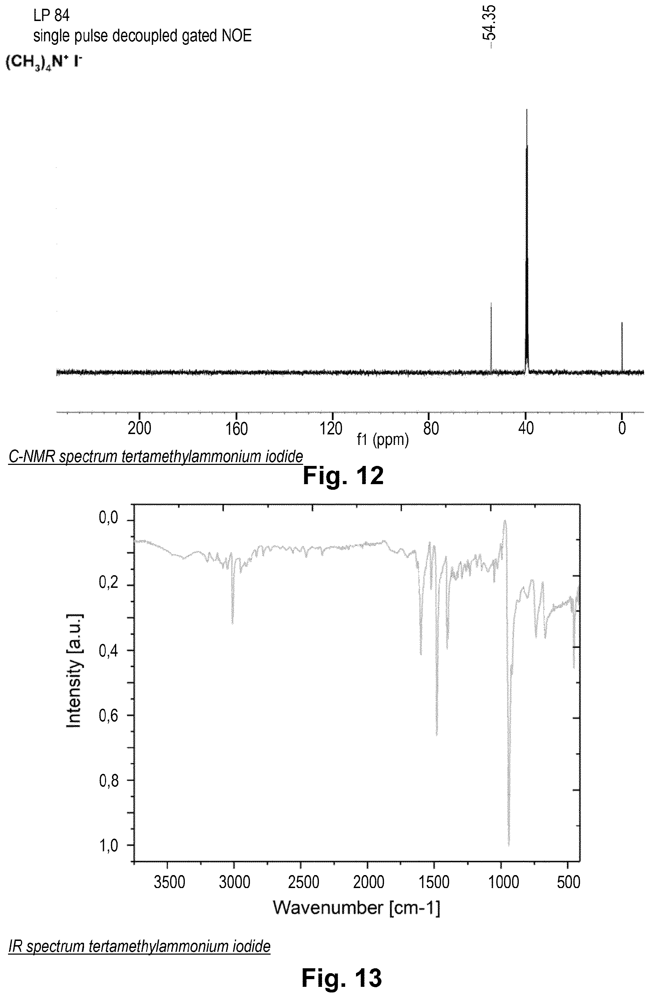

Figs 11, 12, 13, present respective spectra of the obtained porduct (tetramethylammonium iodide).

### EMBODIMENT 8 - synthesis of pyridinium iodide (starting from pyridine):

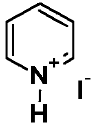

2.10 g of pyridine was weighed and mixed with 10 ml of water. At 10° C, 1.0 ml of formic acid was added into the solution first. Reaction was left at 25 °C for 10 hours. Afterward, 3.36 g of iodine was added. The mixture was agitated for 12 hours at 70° C. The reaction solution was cooled to 25° C. Afterward, 10 ml of ethanol was added, and agitating was performed for 10 minutes, then cold diethyl ether was added. Crystals as products were collected by filtration; the crystals were washed with diethyl ether, and air-dried at 50° C, to afforded 4.60 g of pyridinium iodide (86% yield).

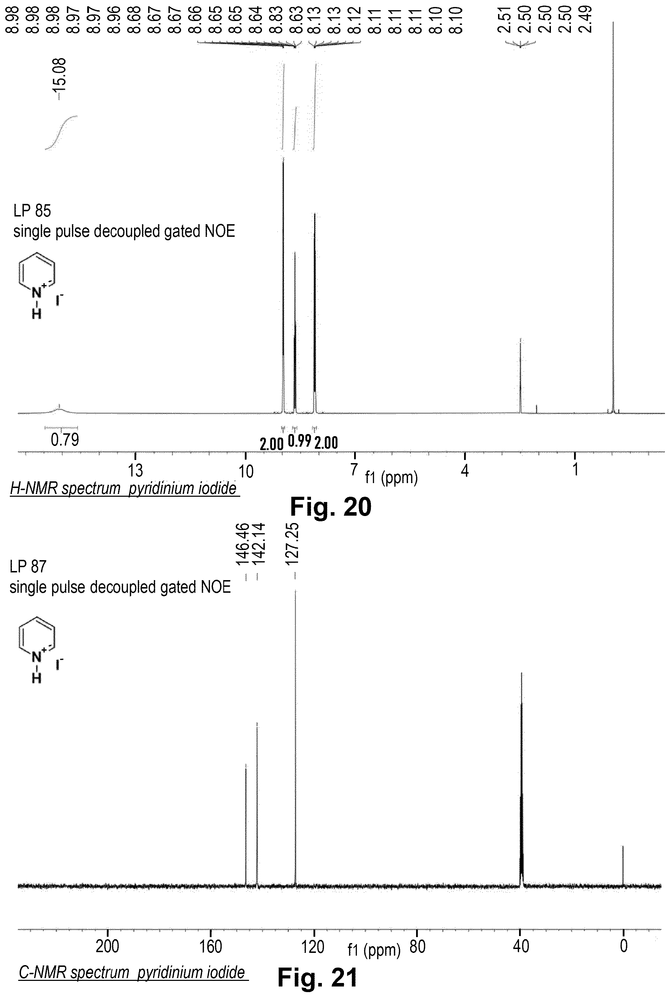

Figs 20, 21, 22 present respective spectra of the obtained product (pyridinium iodide).

### EMBODIMENT 9 - synthesis of imidazolium iodide (starting from imidazole):

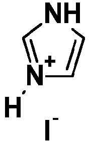

2.70 g of imidazole was weighed and mixed with 12 ml of water. At 10° C, 2.0 ml of formic acid was added into the solution first. Reaction was left at 25 °C for 10 hours. Afterward, 6.72 g of iodine (I₂) was added. The mixture was agitated for 12 hours at 70° C. The reaction solution was cooled to 25° C. Afterward, 15 ml of ethanol was added, and agitating was performed for 10 minutes, then cold diethyl ether was added. Crystals as products were collected by filtration; the crystals were washed with diethyl ether, and air-dried at 50° C, to afforded 9.88 g of imidazolium iodide (95% yield).

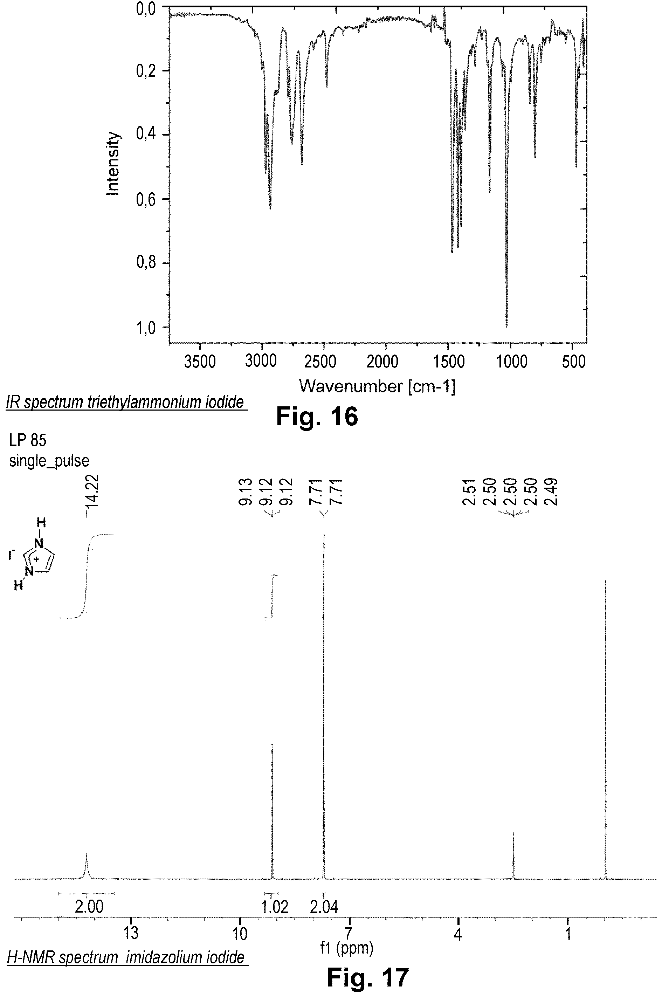

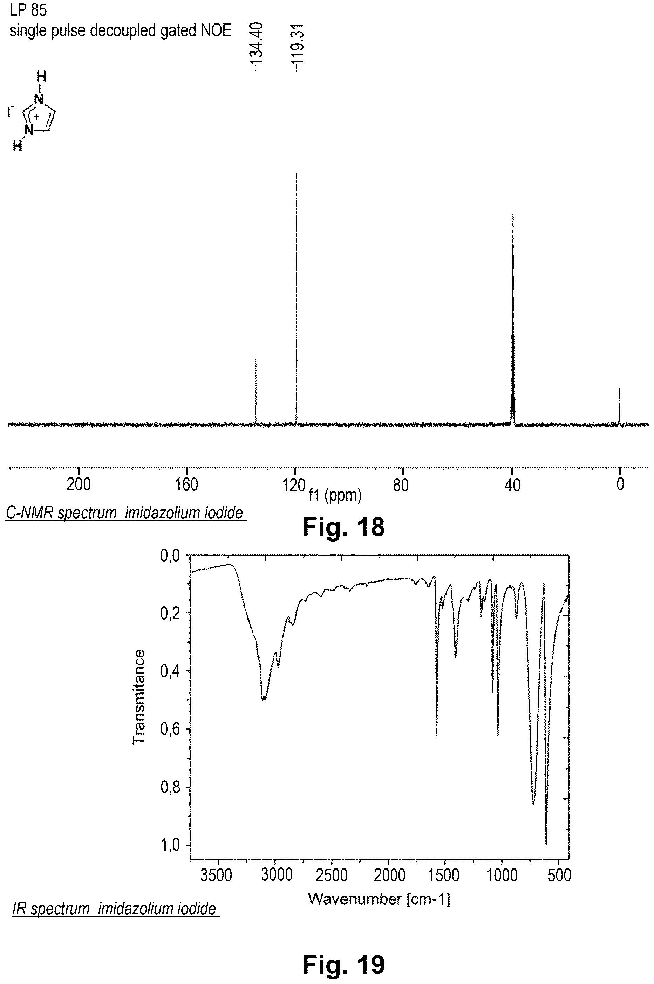

Figs 17, 18, 19 present respective spectra of the obtained product (imidazolium iodide).

### EMBODIMENT 10 - synthesis of triethylammonium iodide (starting from triethylamine):

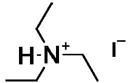

2.14 g of triethylamine was weighed and dissolved in 7 ml of water. At 10° C, 800 ml of formic acid was added into the solution first. Reaction was left at 25 °C for 10 hours. Afterward, 2.69 g of iodine (I₂) was added. The mixture was agitated for 12 hours at 70° C. The reaction solution was cooled to 25° C. Afterward, 9 ml of ethanol was added and agitating was performed for 10 minutes. Crystals as products were collected by filtration; the crystals were washed with diethyl ether, and air-dried at 50° C, to afforded 4.23 g of triethylammonium iodide (87% yield).

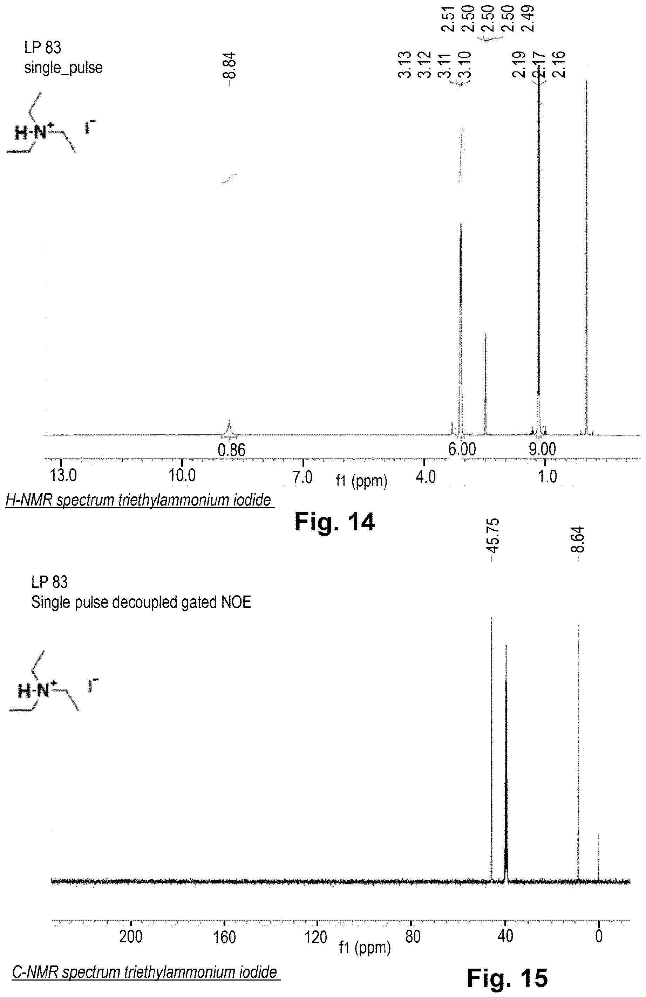

Figs 14, 15, 16 present respective spectra of the obtained product (triethylammonium iodide).

### EMBODIMENT 11 - synthesis of aniline hydroiodide (starting from aniline):

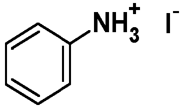

2.47 g of aniline was weighed and dissolved in 8 ml of water. At 10° C, 1 ml of formic acid was added into the solution first. Reaction was left at 25 oC for 10 hours. Afterward, 3.36 g of iodine (I₂) was added. The mixture was agitated for 12 hours at 70° C. The reaction solution was cooled to 25° C. Afterward, 10 ml of ethanol was added and agitating was performed for 10 minutes, then cold diethyl ether was added. Crystals as products were collected by filtration; the crystals were washed with diethyl ether, and air-dried at 50° C, to afforded 3.73 g of aniline hydroiodide (64% yield).

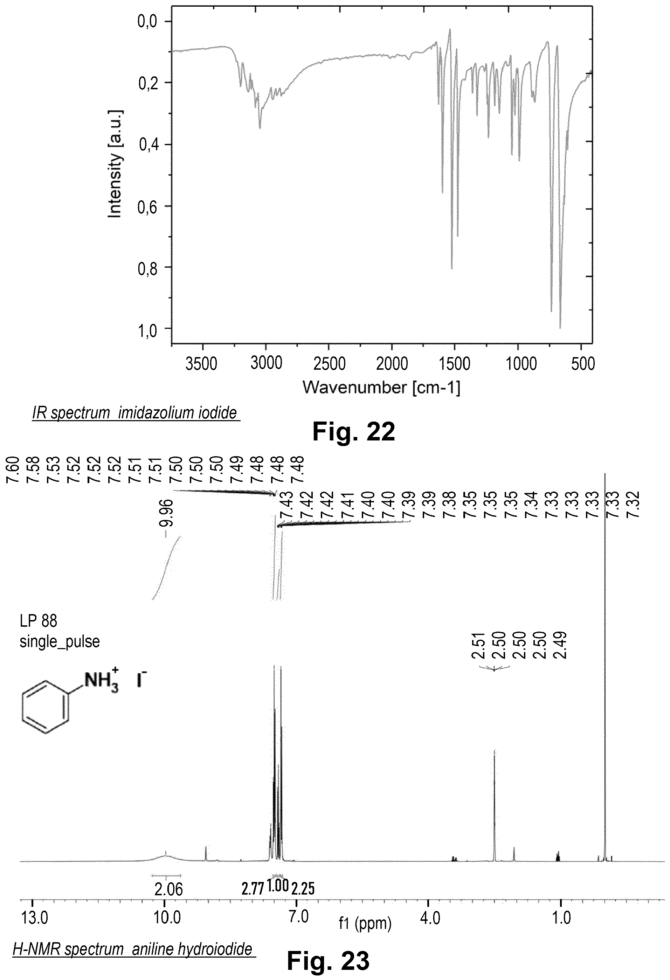

Fig. 23 presents respective spectrum of the obtained product (aniline hydroiodide).

### EMBODIMENT 12 - synthesis of benzylamine hydroiodide (starting from benzylamine):

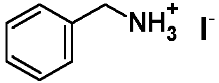

2.13 g of benzylamine was weighted and dissolved in 8.0 ml of water. At 10° C, 750 ml of formic acid was added into the solution first. Reaction was left at 25 °C for 10 hours. Afterward, 2.52 g of iodine (I₂) was added. The mixture was agitated for 12 hours at 70° C. The reaction solution was cooled to 25° C. Afterward, 10 ml of ethanol was added and agitating was performed for 10 minutes, then cold diethyl ether was added. Crystals as products were collected by filtration, washed with diethyl ether, and air-dried at 50° C, to afforded 5.54 g of benzylamine hydroiodide (89% yield).

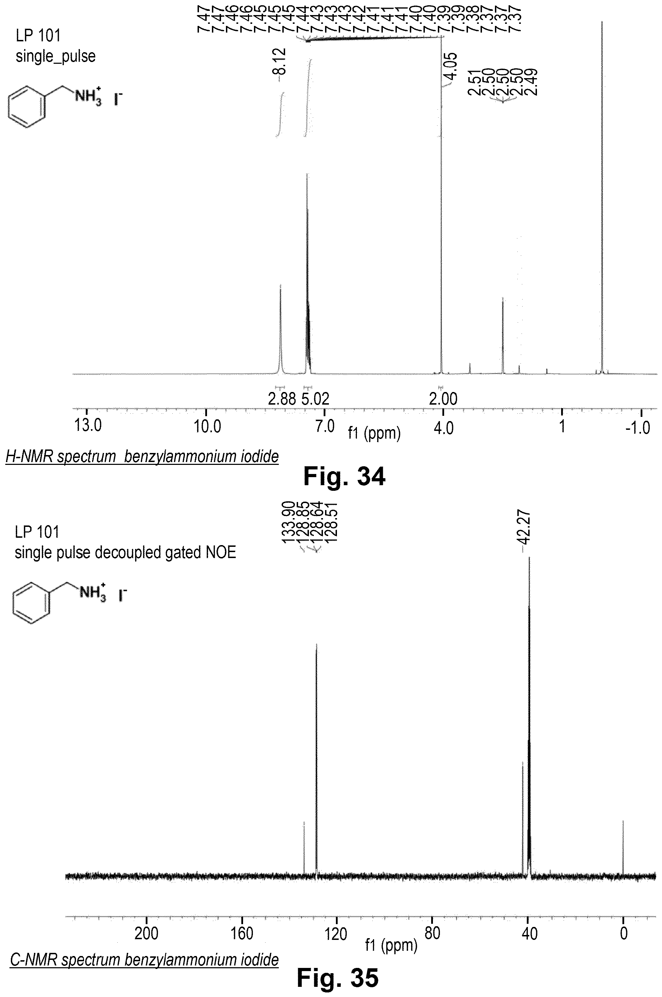

Figs 34, 35, 36 present respective spectra of the obtained product (benzylamine hydroiodide ).

### EMBODIMENT 13 - synthesis of glycine hydroiodide (starting from glycine):

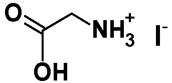

1.50 g of glycine was weighed and dissolved in 16 ml of water. At 50° C, 2.0 ml of formic acid was added into the solution first. Then reaction was left at 50 °C for 10 hours. Afterward, 6.72 g of iodine (I₂) was added. The mixture was agitated for 12 hours at 70° C. The reaction solution was cooled to 25° C. Afterward, 30 ml of ethanol was added and agitating was performed for 10 minutes, then cold diethyl ether was added. Crystals as products were collected by filtration; the crystals were washed with diethyl ether, and air-dried at 50° C, to afforded 4.63 g of glycine hydroiodide (43% yield).

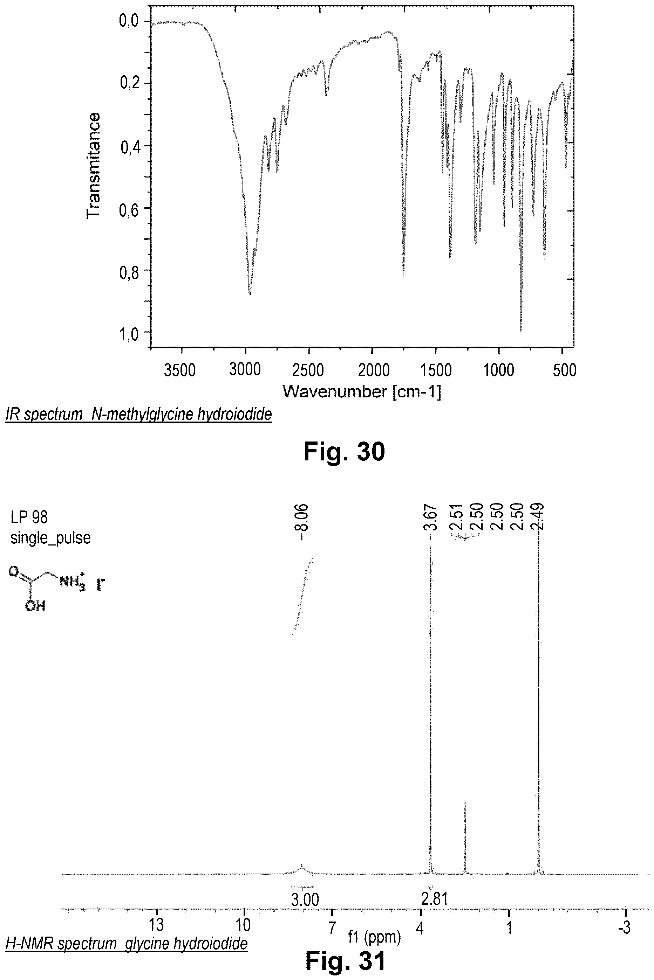

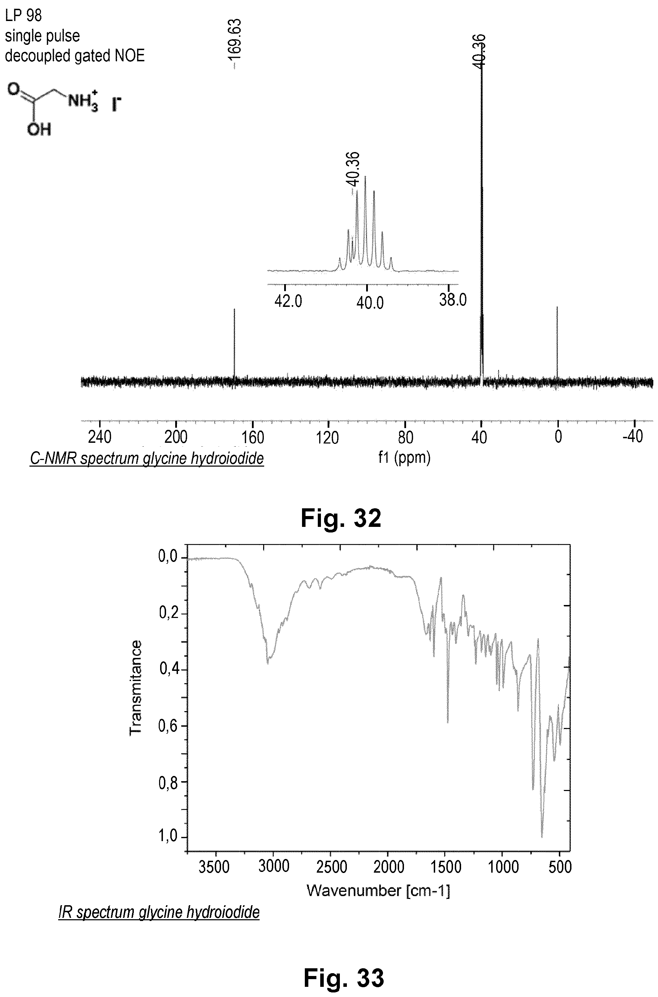

Figs 31, 32, 33 present respective spectra of the obtained product (glycine hydroiodide).

### EMBODIMENT 14 - synthesis of 3-aminopropionic acid hydroiodide (starting from β-alanine):

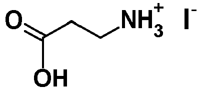

1.78 g of β-alanine was weighed and dissolved in 13 ml of water. At 25° C, 2.0 ml of formic acid was added into the solution first. Reaction was left at 25 °C for 10 hours. Afterward, 6.72 g of iodine was added. The mixture was agitated for 12 hours at 70 °C. The reaction solution was cooled to 25° C. Afterward, 25 ml of ethanol was added, and agitating was performed for 10 minutes, then cold diethyl ether was added. Crystals as products were collected by filtration, washed with diethyl ether, and air-dried at 50° C, to afford 9.68 g of 3-aminopropionic acid hydroiodide (84% yield).

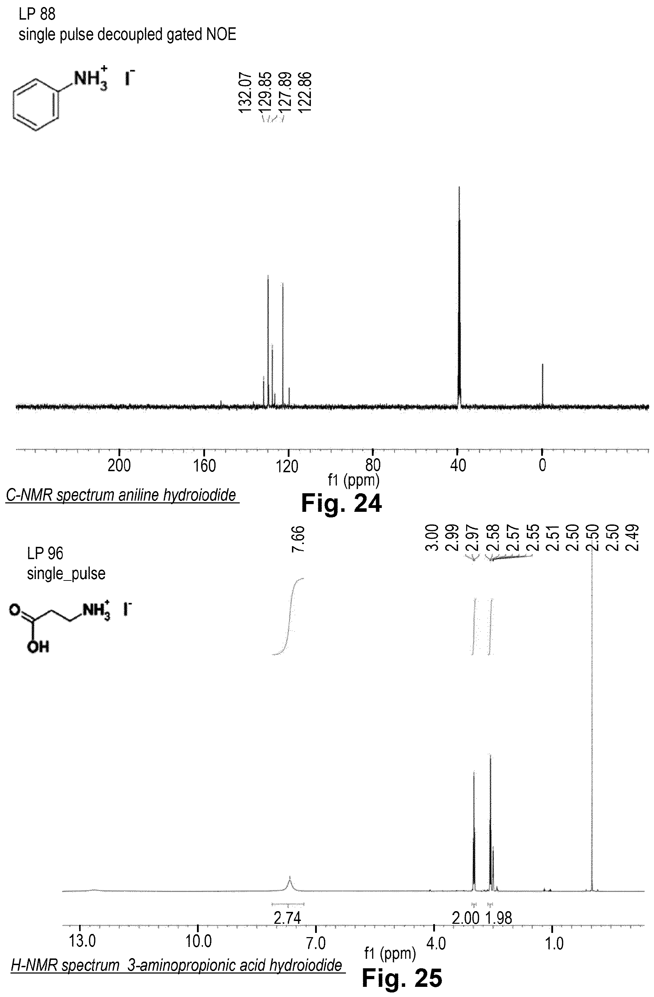

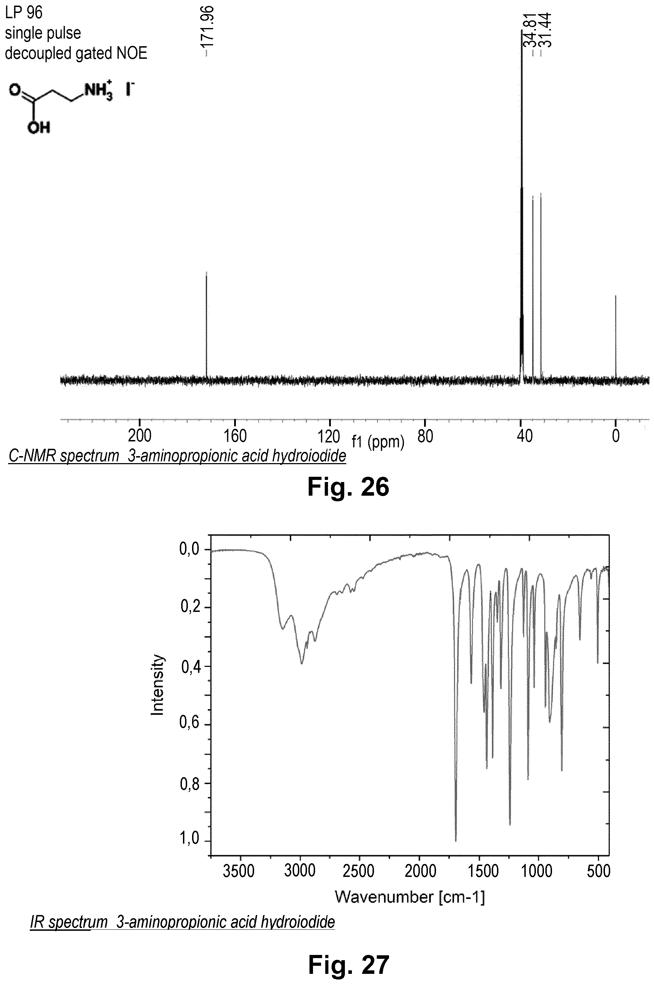

Figs 25, 26, 27 present respective spectra of the obtained product (3-aminopropionic acid hydroiodide).

### EMBODIMENT 15 - synthesis of N-methylglycine hydroiodide (starting from sarcosine):

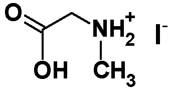

1.78 g of sarcosine was weighed and dissolved in 13 ml of water. At 25 °C, 2.0 ml of formic acid was added into the solution first. Reaction was left at 25 °C for 10 hours. Afterward, 6.72 g of iodine (I₂) was added. The mixture was agitated for 12 hours at 70 °C. The reaction solution was cooled to 25 °C. Afterward, 30 ml of ethanol was added, and agitating was performed for 10 minutes, then cold diethyl ether was added. Crystals as products were collected by filtration, washed with diethyl ether, and air-dried at 50° C, to afford 9.60 g of N-methylglycine hydroiodide (83% yield).

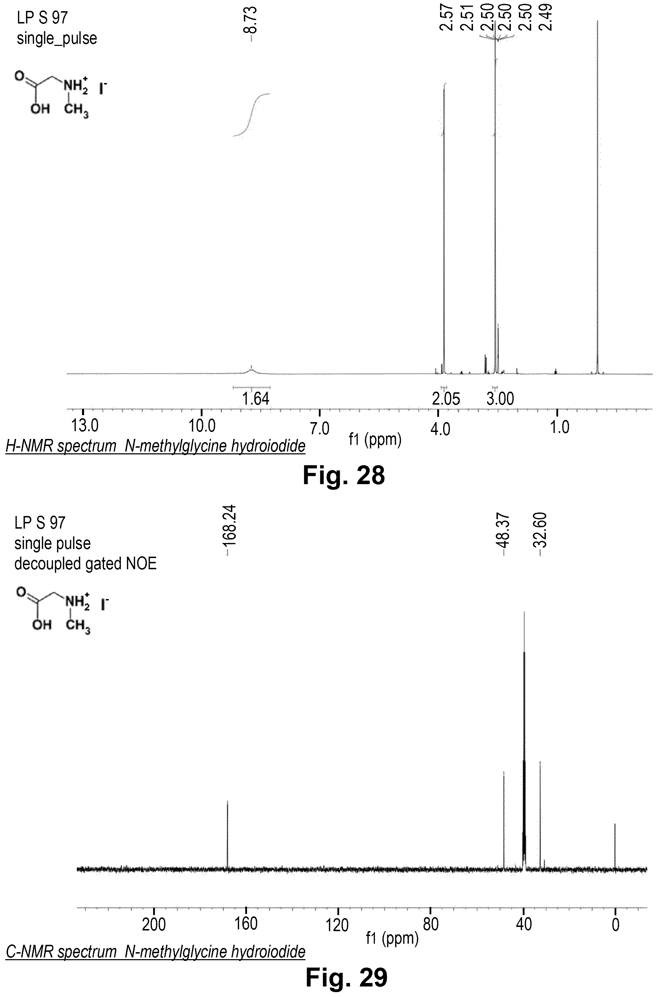

Figs 28, 29, 30 present respective spectra of the obtained product (N-methylglycine hydroiodide).

### EMBODIMENT 16 - synthesis of lead (II) iodide (starting from lead (II) acetate):

5 g lead (II) acetate were dissolved in 10 mL of water. Subsequently, 10 mL of formic acid were added, resulting in a colorless precipitate. The intermediate product was filtrated and washed with cold water. Subsequently, the wet intermediate product was dissolved in 170 mL of hot water. 20 mL of acetone and 4 g of iodine (I₂) were added gradually to the solution under vigorous stirring. The mixture was further agitated for 30 minutes and crystallization of the lead (II) iodide (6 g, 85% yield) was performed in the refrigerator over night.

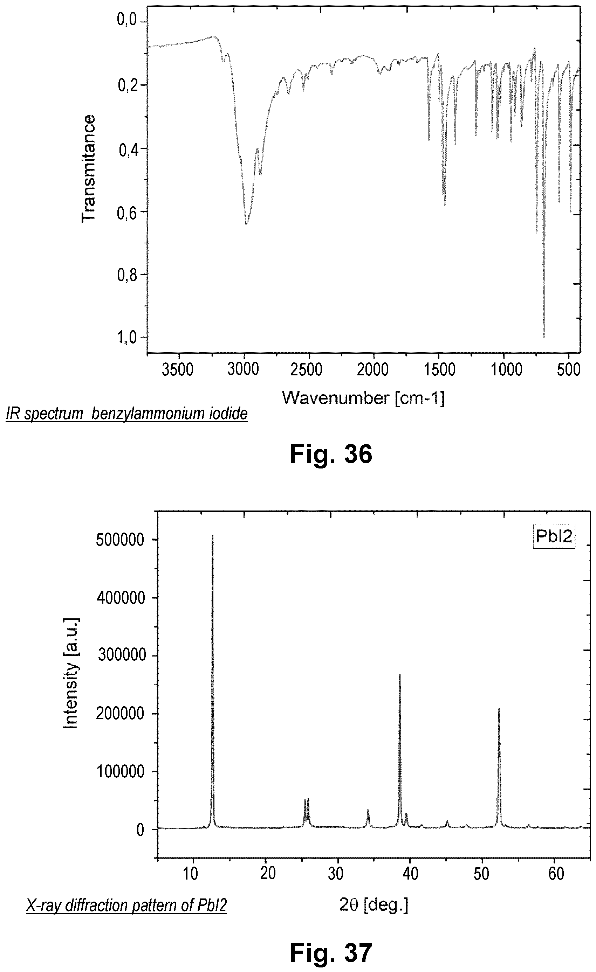

Fig. 37 presents the respective X-ray diffraction pattern of the obtained product (lead (II) iodide).

### EMBODIMENT 17 - synthesis of lead (II) bromide (starting from lead (II) acetate):

5 g lead(II) acetate were dissolved in 10 mL of water. Subsequently, 10 mL of formic acid were added, resulting in a colorless precipitate. The intermediate product was filtrated and washed with cold water. Subsequently, the wet intermediate product was dissolved in 170 mL of hot water. 20 mL of acetone and 0.8 mL of bromine were slowly added to the solution under vigorous stirring. The mixture was further agitated for 30 minutes and crystallization of the colorless lead (II) bromide (3.5 g, 62% yield) was performed in the refrigerator over night.

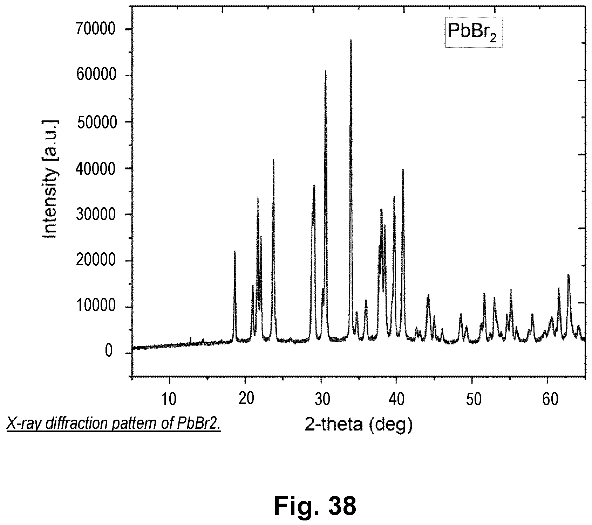

Fig. 38 presents the respective X-ray diffraction pattern of the obtained product (lead (II) bromide).

## Claims

1. A method for synthesis of halide salts of the general formula (I):
**Zⁿ⁺X⁻ₙ** **Formula I**
wherein:
X⁻ represents a halogen anion, and
Zⁿ⁺ represents a cation having a valency n,
the method comprising reacting a first reactant being formate salt of the general formula (II):
**Zⁿ⁺(COOH⁻)ₙ** **Formula II**
with a second reactant being a diatom halogen: X₂.

2. The method according to claim 1 wherein said diatom halogen X₂ is selected from Br₂ and I₂.

3. The method according to any of the preceding claims wherein said cation (Zⁿ⁺) is an inorganic cation of a metal selected from the group consisting of Cs⁺, Ag⁺, Pb²⁺, Sn²⁺, Bi³⁺, and Sn⁴⁺.

4. The method according to any of the preceding claims wherein said cation (Zⁿ⁺) is Pb²⁺.

5. The method according to claim 1 or 2 wherein said cation (Zⁿ⁺) is an organic cation of the general formula (III), (IV), or (V): 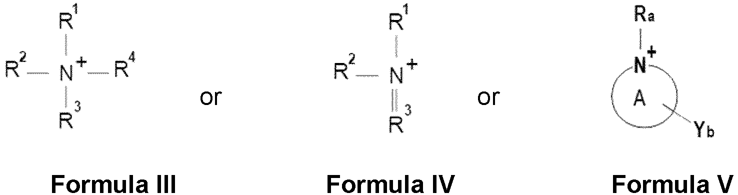 wherein:
**R, R¹, R², R³, R⁴** each independently represents hydrogen or an organic substituent moiety, wherein at least one of R¹, R², R³ is not hydrogen;
**a** denotes the number of R moieties,
**A** represents five-membered or six-membered aliphatic or aromatic hydrocarbon ring, optionally having at least one heteroatom substituting the carbon atom selected from oxygen, nitrogen, and sulphur,
**Y** represents a substituent of the ring A other than hydrogen.
**b** - denotes the number of Y moieties, wherein b is zero or an integer in the range of 1 to 4 if A represents the five-membered ring, or b is zero or an integer in the range of 1 to 5 if A represents the six-membered ring.

6. The method according to claim 5 wherein the organic substituent independently for each of R, R¹, R², R³, and R⁴, is selected from C₁-C₁₆ alkyl, C₆ aryl or C₁₀ aryl, C₇₋₂₆ aralkyl, or heterocycle substituent.

7. The method according to claim 5 or 6 wherein the organic substituent independently for each of R, R¹, R², R³, and R⁴, is selected from:
C₁-C₁₆ alkyl constituting acyclic, straight or branched hydrocarbon chain comprising from 1 to 16 carbon atoms in the chain, optionally comprising at least one heteroatom substituting the carbon atom or hydrogen atom in the C₁-C₁₆ alkyl moiety, wherein the heteroatom being selected from the group consisting of phosphorus, silicon, oxygen, nitrogen, sulphur, iodine, chlorine, bromine, and fluorine;
C₆ aryl or C₁₀ aryl constituting an aromatic monocyclic system comprising six carbon atoms forming the ring system, or an aromatic bicyclic system comprising ten carbon atoms forming the bicyclic system, optionally comprising at least one heteroatom substituting the carbon atom or hydrogen atom in the C₆ aryl or C₁₀ aryl moiety, wherein the heteroatom is selected from the group consisting of phosphorus, silicon, oxygen, nitrogen, sulphur, iodine, chlorine, bromine, and fluorine;
C₇₋₂₆ aralkyl constituting the C₆ arlyl or C₁₀ aryl substituent linked to the nitrogen atom of the organic cation through the C₁-C₁₆ alkyl, optionally comprising at least one heteroatom substituting the carbon atom or hydrogen atom, wherein the heteroatom is selected from the group consisting of: phosphorus, silicon, oxygen, nitrogen, sulphur, iodine, chlorine, bromine and fluorine; and
heterocycle substituent constituting monovalent substituent derived by removing hydrogen from a five-, six-, or seven-membered saturated or unsaturated alicyclic moiety or aromatic heterocycle moiety, comprising carbon atoms and from one to four ring heteroatoms selected from nitrogen, oxygen, and sulfur.

8. The method according to any of the claims from 5 to 7 wherein the organic substituent independently for each of R, R¹, R², R³ and R⁴, is selected from:
C₁-C₁₆ alkyl substituent selected from the group consisting of methyl, ethyl, propyl, isopropyl n-butyl, tert-butyl, isobutyl, sec-butyl, n-pentyl, isopenthyl, neopenthyl, methylethyl, diispropylethyl, 2-methylhexyl, 3-methylheptyl, 2,3-dimethylpentyl, 4-ethyl-2-methyloctyl, 4-isopropylnonyl, hexyl, 2-methylpentyl, 3-methylpentyl, 2,2-dimethylbutyl, and 2,3-dimethylbutyl, 1-methylhexyl, 2-methylhexyl, 1,1-dimethylpentyl, 1,2- dimethylpentyl, 1,3- dimethylpentyl, 2,2-dimethylpentyl, 3-ethylpentyl, 2,2,3-trimethylbutyl, 2-methylheptyl, 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, 2,2,4-trimethylpentyl, 2,3,3-Trimethylpentyl, 3,3,4-trimethyl-pentyl, 3,4,4-trimethylpentyl, 2,4,4-trimethylpentyl, 2-methylheptyl, 3-methylheptyl, 2,3-dimethylhexyl, 3,4-dimethylhexyl, 2,3,4-trimethylpentyl, 3,3-dimethylhexyl, 2,2-trimethylpentyl, 2,4-dimethylhexyl, optionally comprising at least one heteroatom substituting the carbon atom or hydrogen atom in the C₁-C₁₆ alkyl moiety, wherein the heteroatom being selected from the group consisting of phosphorus, silicon, oxygen, nitrogen, sulphur, iodine, chlorine, bromine, and fluorine;
C₆ aryl or C₁₀ aryl substituent selected from the group consisting of phenyl and naphthyl, optionally comprising at least one heteroatom substituting the carbon atom or hydrogen atom in the C₆ or C₁₀ aryl moiety, wherein the heteroatom is selected from the group consisting of: phosphorus, silicon, oxygen, nitrogen, sulphur, iodine, chlorine, bromine, and fluorine;
C₇₋₂₆ aralkyl substituent selected from the group consisting of benzyl, butylphenyl, ethylphenyl, propylphenyl, pentylphenyl, hexylphenyl, ethylnaphtyl, and methylnaphtyl, optionally comprising at least one heteroatom substituting the carbon atom or hydrogen atom, wherein the heteroatom is selected from the group consisting of phosphorus, silicon, oxygen, nitrogen, sulphur, iodine, chlorine, bromine and fluorine; and
heterocycle substituent selected from the group consisting of furan, tetrahydrofuran, thiophene, diazepine, isoxazole, piperidine, dioxane, pyridine, and pyrimidine.

9. The method according to any of the claim from 5 to 8 wherein said cation (Zⁿ⁺) is the organic cation of the formula (VI), (VII), or (VIII):

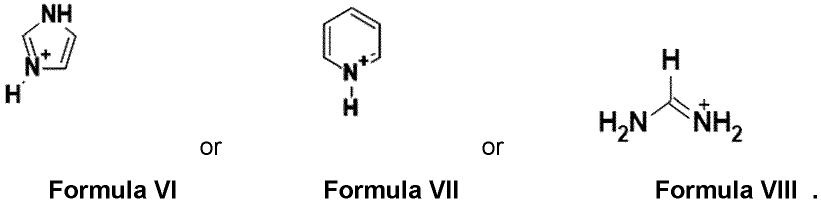

10. The method according to any of the preceding claims comprising heating the reactants to the temperature of 25 to 130°C until obtaining the halide salt.

11. The method according to any of the preceding claims wherein reacting of the reactants is carried out in the absence of a solvent.

12. The method according to any of the claims from 1 to 10 wherein reacting of the reactants is carried out in the presence of a solvent.

13. The method according to claims 3 or 4 and 12 wherein the solvent is selected from the group consisting of water and acetone.

14. The method according to any of the claims from 5 to 9 and claim 12 wherein the solvent is selected from the group consisting of water, alcohols, and acetonitriles.

15. The method according to any of the preceding claims further comprising recrystallizing the obtained halide salt.

## Patentansprüche

1. Verfahren zur Synthese von Halogenidsalzen der allgemeinen Formel (I):
**Zⁿ⁺X⁻ₙ** **Formel I**
wobei:
X⁻ ein Halogenanion darstellt, und
Zⁿ⁺ ein Kation darstellt, das eine Wertigkeit n aufweist,
das Verfahren umfassend ein Reagieren eines ersten Reaktanten, der ein Formiatsalz der allgemeinen Formel (II) ist:
**Zⁿ⁺(COOH⁻)ₙ** **Formel II**
wobei ein zweiter Reaktant ein Diatomeenhalogen ist: X₂.

2. Verfahren nach Anspruch 1, wobei das Diatomeenhalogen X₂ ausgewählt ist aus Br₂ und I₂.

3. Verfahren nach einem der vorhergehenden Ansprüche, wobei das Kation (Zⁿ⁺) ein anorganisches Kation eines Metalls ist, das ausgewählt ist aus der Gruppe, bestehend aus Cs⁺, Ag⁺, Pb²⁺, Sn²⁺, Bi³⁺ und Sn⁴⁺.

4. Verfahren nach einem der vorhergehenden Ansprüche, wobei das Kation (Zⁿ⁺) Pb²⁺ ist.

5. Verfahren nach Anspruch 1 oder 2, wobei das Kation (Zⁿ⁺) ein organisches Kation der allgemeinen Formel (III), (IV) oder (V) ist: 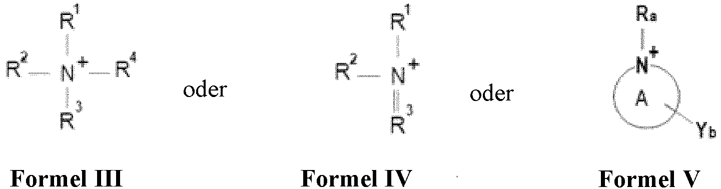 wobei:
**R, R¹, R², R³, R⁴** jeweils unabhängig Wasserstoff oder eine organische Substituentengruppierung darstellen, wobei mindestens eines von R¹, R², R³ nicht Wasserstoff ist;
**a** die Anzahl von R-Gruppierungen bezeichnet,
**A** fünfgliedrigen oder sechsgliedrigen aliphatischen oder aromatischen Kohlenwasserstoffring darstellt, der optional mindestens ein Heteroatom aufweist, das das Kohlenstoffatom substituiert, das ausgewählt ist aus Sauerstoff, Stickstoff und Schwefel,
**Y** einen anderen Substituenten des Rings A als Wasserstoff darstellt
**b** - die Anzahl von Y-Gruppierungen bezeichnet, wobei b null oder eine ganze Zahl in dem Bereich von 1 bis 4 ist, wenn A den fünfgliedrigen Ring darstellt, oder b null oder eine ganze Zahl in dem Bereich von 1 bis 5 ist, wenn A den sechsgliedrigen Ring darstellt.

6. Verfahren nach Anspruch 5, wobei der organische Substituent unabhängig für jedes von R, R¹, R², R³ und R⁴ ausgewählt ist aus C₁-C₁₆-Alkyl, C₆-Aryl oder C₁₀-Aryl, C₇₋₂₆-Aralkyl oder Heterocyclus-Substituent.

7. Verfahren nach Anspruch 5 oder 6, wobei der organische Substituent unabhängig für jedes von R, R¹, R², R³ und R⁴ ausgewählt ist aus:
C₁-C₁₆-Alkyl, das eine acyclische, geradkettige oder verzweigte Kohlenwasserstoffkette bedeutet, umfassend 1 bis 16 Kohlenstoffatome in der Kette, optional umfassend mindestens ein Heteroatom, das das Kohlenstoffatom oder Wasserstoffatom in der C₁-C₁₆-Alkylgruppierung substituiert, wobei das Heteroatom ausgewählt ist aus der Gruppe, bestehend aus Phosphor, Silicium, Sauerstoff, Stickstoff, Schwefel, Jod, Chlor, Brom und Fluor;
C₆-Aryl oder C₁₀-Aryl die ein aromatisches monocyclisches System bedeuten, umfassend sechs Kohlenstoffatome, die das Ringsystem bilden, oder ein aromatisches bicyclisches System, umfassend zehn Kohlenstoffatome, die das bicyclische System bilden, optional umfassend mindestens ein Heteroatom, das das Kohlenstoffatom oder Wasserstoffatom in der C₆-Aryl- oder C₁₀-Arylgruppierung substituiert, wobei das Heteroatom ausgewählt ist aus der Gruppe, bestehend aus Phosphor, Silicium, Sauerstoff, Stickstoff, Schwefel, Jod, Chlor, Brom und Fluor;
C₇₋₂₆-Aralkyl, das den C₆-Aryl- oder C₁₀-Arylsubstituenten bedeutet, der über das C₁-C₁₆-Alkyl an das Stickstoffatom des organischen Kations gebunden ist, optional umfassend mindestens ein Heteroatom, das das Kohlenstoffatom oder Wasserstoffatom substituiert, wobei das Heteroatom ausgewählt ist aus der Gruppe, bestehend aus: Phosphor, Silicium, Sauerstoff, Stickstoff, Schwefel, Jod, Chlor, Brom und Fluor; und
Heterocyclus-Substituent, der einen einwertigen Substituenten bedeutet, der durch Entfernen von Wasserstoff von einer fünf-, sechs- oder siebengliedrigen gesättigten oder ungesättigten alicyclischen Gruppierung oder einer aromatischen Heterocyclus-Gruppierung abgeleitet ist, umfassend Kohlenstoffatome und ein bis vier Ringheteroatome, ausgewählt aus Stickstoff, Sauerstoff und Schwefel.

8. Verfahren nach einem der Ansprüche 5 bis 7, wobei der organische Substituent unabhängig für jedes von R, R¹, R², R³ und R⁴ ausgewählt ist aus:
C₁-C₁₆-Alkylsubstituent, ausgewählt aus der Gruppe bestehend aus Methyl, Ethyl, Propyl, Isopropyl, n-Butyl, tert-Butyl, Isobutyl, sec-Butyl, n-Pentyl, Isopenthyl, Neopenthyl, Methylethyl, Diispropylethyl, 2-Methylhexyl, 3-Methylheptyl, 2,3-Dimethylpentyl, 4-Ethyl-2-Methyloctyl, 4-Isopropylnonyl, Hexyl, 2-Methylpentyl, 3-Methylpentyl, 2,2-Dimethylbutyl und 2,3-Dimethylbutyl, 1-Methylhexyl, 2-Methylhexyl, 1,1-Dimethylpentyl, 1,2-Dimethylpentyl, 1,3-Dimethylpentyl, 2,2-Dimethylpentyl, 3-Ethylpentyl, 2,2,3-Trimethylbutyl, 2-Methylheptyl, 3-Methylheptyl, 2,3-Dimethylhexyl, 3,4-Dimethylhexyl, 2,3,4-Trimethylpentyl, 3,3-Dimethylhexyl, 2,2-Trimethylpentyl, 2,4-Dimethylhexyl, 2,2,4-Trimethylpentyl, 2,3,3-Trimethylpentyl, 3,3,4-Trimethylpentyl, 3,4,4-Trimethylpentyl, 2,4,4-Trimethylpentyl, 2-Methylheptyl, 3-Methylheptyl, 2,3-Dimethylhexyl, 3,4-Dimethylhexyl, 2,3,4-Trimethylpentyl, 3,3-Dimethylhexyl, 2,2-Trimethylpentyl, 2,4-Dimethylhexyl, optional umfassend mindestens ein Heteroatom, das das Kohlenstoffatom oder Wasserstoffatom in der C₁-C₁₆-Alkylgruppierung substituiert, wobei das Heteroatom ausgewählt ist aus der Gruppe, bestehend aus Phosphor, Silicium, Sauerstoff, Stickstoff, Schwefel, Jod, Chlor, Brom und Fluor;
C₆-Aryl oder C₁₀-Arylsubstituent, ausgewählt aus der Gruppe, bestehend aus Phenyl und Naphthyl, optional umfassend mindestens ein Heteroatom, das das Kohlenstoffatom oder Wasserstoffatom in der C₆- oder C₁₀-Arylgruppierung substituiert, wobei das Heteroatom ausgewählt ist aus der Gruppe, bestehend aus: Phosphor, Silicium, Sauerstoff, Stickstoff, Schwefel, Jod, Chlor, Brom und Fluor;
C₇₋₂₆-Aralkylsubstituent, ausgewählt aus der Gruppe, bestehend aus Benzyl, Butylphenyl, Ethylphenyl, Propylphenyl, Pentylphenyl, Hexylphenyl, Ethylnaphtyl und Methylnaphtyl, optional umfassend mindestens ein Heteroatom, das das Kohlenstoffatom oder Wasserstoffatom substituiert, wobei das Heteroatom ausgewählt ist aus der Gruppe, bestehend aus Phosphor, Silicium, Sauerstoff, Stickstoff, Schwefel, Jod, Chlor, Brom und Fluor; und
Heterocyclus-Substituent, ausgewählt aus der Gruppe, bestehend aus Furan, Tetrahydrofuran, Thiophen, Diazepin, Isoxazol, Piperidin, Dioxan, Pyridin und Pyrimidin.

9. Verfahren nach einem der Ansprüche 5 bis 8, wobei das Kation (Zⁿ⁺) das organische Kation der Formel (VI), (VII) oder (VIII) ist:

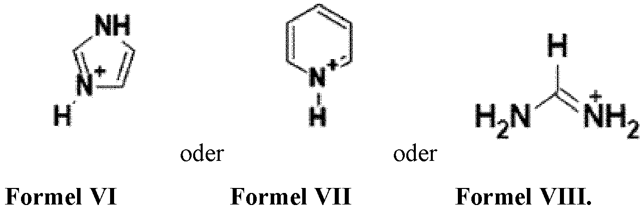

10. Verfahren nach einem der vorhergehenden Ansprüche umfassend ein Erhitzen der Reaktanten auf die Temperatur von 25 bis 130°C, bis das Halogenidsalz erlangt wird.

11. Verfahren nach einem der vorhergehenden Ansprüche, wobei ein Reagieren der Reaktanten in Abwesenheit eines Lösungsmittels durchgeführt wird.

12. Verfahren nach einem der Ansprüche 1 bis 10, wobei das Reagieren der Reaktanten in Anwesenheit eines Lösungsmittels durchgeführt wird.

13. Verfahren nach einem der Ansprüche 3 oder 4 und 12, wobei das Lösungsmittel ausgewählt ist aus der Gruppe, bestehend aus Wasser und Aceton.

14. Verfahren nach einem der Ansprüche 5 bis 9 und Anspruch 12, wobei das Lösungsmittel ausgewählt ist aus der Gruppe bestehend aus Wasser, Alkoholen und Acetonitrilen.

15. Verfahren nach einem der vorhergehenden Ansprüche, ferner umfassend ein Umkristallisieren des erlangten Halogenidsalzes.

## Revendications

1. Procédé de synthèse de sels d'halogénure de la formule générale (I) :
**Zⁿ⁺X⁻ₙ** **Formule I**
où :
X⁻ représente un anion halogène, et
Zⁿ⁺ représente un cation possédant une valence n,
le procédé comprenant la mise en réaction d'un premier réactif qui est un sel de formiate de la formule générale (II) :
**Zⁿ⁺(COOH⁻)ₙ** **Formule II**
avec un second réactif qui est un halogène diatomique : X₂.

2. Procédé selon la revendication 1 dans lequel ledit halogène diatomique X₂ est choisi parmi Br₂ et I₂.

3. Procédé selon l'une quelconque des revendications précédentes, dans lequel ledit cation (Zⁿ⁺) est un cation inorganique d'un métal choisi dans le groupe constitué de Cs⁺, Ag⁺, Pb²⁺, Sn²⁺, Bi³⁺ et Sn⁴⁺.

4. Procédé selon l'une quelconque des revendications précédentes, dans lequel ledit cation (Zⁿ⁺) est Pb²⁺.

5. Procédé selon l'une des revendications 1 ou 2, dans lequel ledit cation (Zⁿ⁺) est un cation organique de la formule générale (III), (IV) ou (V) : 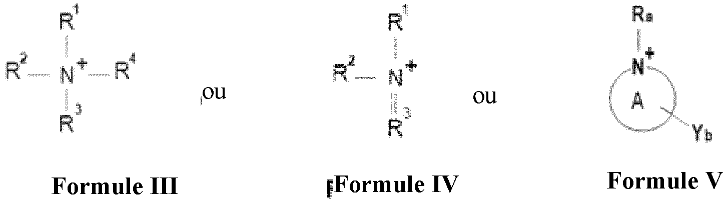 où :
**R, R¹, R², R³, R⁴** représentent chacun indépendamment un hydrogène ou un groupement substituant organique, au moins l'un de R¹, R², R³ n'étant pas un hydrogène ;
**a** désigne le nombre de groupements R,
**A** représente un cycle hydrocarboné aliphatique ou aromatique à cinq ou six chaînons, comportant éventuellement au moins un hétéroatome substituant l'atome de carbone choisi parmi l'oxygène, l'azote et le soufre,
**Y** représente un substituant du cycle A autre que l'hydrogène,
**b** - désigne le nombre de groupements Y, où b vaut zéro ou est un entier compris dans la plage de 1 à 4 si A représente le cycle à cinq chaînons, ou b vaut zéro ou est un entier compris dans la plage de 1 à 5 si A représente le cycle à six chaînons.

6. Procédé selon la revendication 5, dans lequel le substituant organique, indépendamment de chacun de R, R¹, R², R³ et R⁴, est choisi parmi un alkyle en C₁-C₁₆, un aryle en C₆ ou un aryle en C₁₀, un aralkyle en C₇₋₂₆ ou un substituant hétérocyclique.

7. Procédé selon la revendication 5 ou 6, dans lequel le substituant organique indépendamment de chacun de R, R¹, R², R³ et R⁴, est choisi parmi :
un alkyle en C₁-C₁₆ constituant une chaîne hydrocarbonée acyclique, droite ou ramifiée comprenant 1 à 16 atomes de carbone dans la chaîne, comprenant éventuellement au moins un hétéroatome substituant l'atome de carbone ou l'atome d'hydrogène dans le groupement alkyle en C₁-C₁₆, l'hétéroatome étant choisi dans le groupe constitué par le phosphore, le silicium, l'oxygène, l'azote, le soufre, l'iode, le chlore, le brome et le fluor ;
un aryle en C₆ ou un aryle en C₁₀ constituant un système monocyclique aromatique comprenant six atomes de carbone formant le système cyclique, ou un système bicyclique aromatique comprenant dix atomes de carbone formant le système bicyclique, comprenant éventuellement au moins un hétéroatome substituant l'atome de carbone ou l'atome d'hydrogène dans le groupement aryle en C₆ ou aryle en C₁₀, l'hétéroatome étant choisi dans le groupe constitué par le phosphore, le silicium, l'oxygène, l'azote, le soufre, l'iode, le chlore, le brome et le fluor ;
un aralkyle en C₇₋₂₆ constituant le substituant aryle en C₆ ou aryle en C₁₀ lié à l'atome d'azote du cation organique par l'alkyle en C₁-C₁₆, comprenant éventuellement au moins un hétéroatome substituant l'atome de carbone ou l'atome d'hydrogène, l'hétéroatome étant choisi dans le groupe constitué par : le phosphore, le silicium, l'oxygène, l'azote, le soufre, l'iode, le chlore, le brome et le fluor ; et
un substituant hétérocyclique constituant un substituant monovalent dérivé par élimination d'hydrogène d'un groupement alicyclique saturé ou insaturé à cinq, six ou sept chaînons ou d'un groupement hétérocyclique aromatique, comprenant des atomes de carbone et un à quatre hétéroatomes cycliques choisis parmi l'azote, l'oxygène et le soufre.

8. Procédé selon l'une quelconque des revendications 5 à 7, dans lequel le substituant organique indépendamment de chacun de R, R¹, R², R³ et R⁴, est choisi parmi :
un substituant alkyle en C₁-C₁₆ choisi dans le groupe constitué par un méthyle, un éthyle, un propyle, un isopropyle, un n-butyle, un tert-butyle, un isobutyle, un sec-butyle, un n-pentyle, un isopentyle, un néopentyle, un méthyléthyle, un diisopropyléthyle, un 2-méthylhexyle, un 3-méthylheptyle, un 2,3-diméthylpentyle, un 4-éthyl-2-méthyloctyle, un 4-isopropylnonyle, un hexyle, un 2-méthylpentyle, un 3-méthylpentyle, un 2,2-diméthylbutyle et un 2,3-diméthylbutyle, un 1-méthylhexyle, un 2-méthylhexyle, un 1,1-diméthylpentyle, un 1,2-diméthylpentyle, un 1,3-diméthylpentyle, un 2,2-diméthylpentyle, un 3-éthylpentyle, un 2,2,3-triméthylbutyle, un 2-méthylheptyle, un 3-méthylheptyle, un 2,3-diméthylhexyle, un 3,4-diméthylhexyle, un 2,3,4-triméthylpentyle, un 3,3-diméthylhexyle, un 2,2-triméthylpentyle, un 2,4-diméthylhexyle, un 2,2,4-triméthylpentyle, un 2,3,3-triméthylpentyle, un 3,3,4-triméthylpentyle, un 3,4,4-triméthylpentyle, un 2,4,4-triméthylpentyle, un 2-méthylheptyle, un 3-méthylheptyle, un 2,3-diméthylhexyle, un 3,4-diméthylhexyle, un 2,3,4-triméthylpentyle, un 3,3-diméthylhexyle, un 2,2-triméthylpentyle, un 2,4-diméthylhexyle, comprenant éventuellement au moins un hétéroatome substituant l'atome de carbone ou l'atome d'hydrogène dans le groupement alkyle en C₁-C₁₆, l'hétéroatome étant choisi dans le groupe constitué par le phosphore, le silicium, l'oxygène, l'azote, le soufre, l'iode, le chlore, le brome et le fluor ;
un substituant aryle en C₆ ou aryle en C₁₀ choisi dans le groupe constitué par un phényle et un naphtyle, comprenant éventuellement au moins un hétéroatome substituant l'atome de carbone ou l'atome d'hydrogène dans le groupement aryle en C₆ ou C₁₀, l'hétéroatome étant choisi dans le groupe constitué par : le phosphore, le silicium, l'oxygène, l'azote, le soufre, l'iode, le chlore, le brome et le fluor ;
un substituant aralkyle en C₇₋₂₆ choisi dans le groupe constitué par un benzyle, un butylphényle, un éthylphényle, un propylphényle, un pentylphényle, un hexylphényle, un éthylnaphtyle et un méthylnaphtyle, comprenant éventuellement au moins un hétéroatome substituant l'atome de carbone ou l'atome d'hydrogène, l'hétéroatome étant choisi dans le groupe constitué par le phosphore, le silicium, l'oxygène, l'azote, le soufre, l'iode, le chlore, le brome et le fluor ; et
un substituant hétérocyclique choisi dans le groupe constitué par un furane, un tétrahydrofurane, un thiophène, une diazépine, un isoxazole, une pipéridine, un dioxane, une pyridine et une pyrimidine.

9. Procédé selon l'une quelconque des revendications 5 à 8, dans lequel ledit cation (Zⁿ⁺) est le cation organique de la formule (VI), (VII) ou (VIII) :

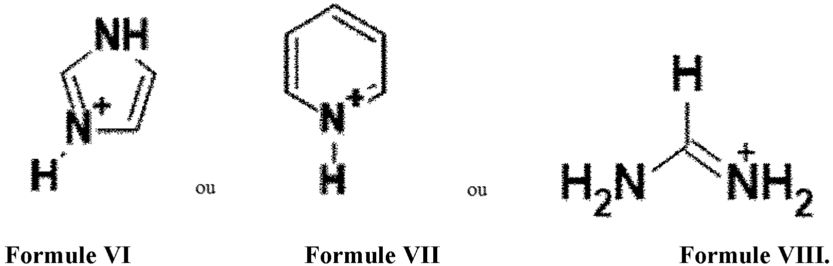

10. Procédé selon l'une quelconque des revendications précédentes, comprenant le chauffage des réactifs à une température de 25 à 130 °C jusqu'à l'obtention du sel d'halogénure.

11. Procédé selon l'une quelconque des revendications précédentes, dans lequel la réaction des réactifs est effectuée en l'absence de solvant.

12. Procédé selon l'une quelconque des revendications 1 à 10, dans lequel la réaction des réactifs est effectuée en présence d'un solvant.

13. Procédé selon les revendications 3 ou 4 et 12, dans lequel le solvant est choisi dans le groupe constitué par l'eau et l'acétone.

14. Procédé selon l'une quelconque des revendications 5 à 9 et la revendication 12, dans lequel le solvant est choisi dans le groupe constitué par l'eau, les alcools et les acétonitriles.

15. Procédé selon l'une quelconque des revendications précédentes, comprenant en outre la recristallisation du sel d'halogénure obtenu.
